# EUROPEAN PATENT SPECIFICATION

(11) **EP 3 274 003 B1**
(45) Date of publication and mention of the grant of the patent: **17.07.2019**
(21) Application number: 16714296.7
(22) Date of filing: 18.03.2016
(51) Int. Cl.: A61L 27/42, A61L 27/54, A61L 27/58, A61L 27/50

(54) **BIPHASIC CERAMIC BONE SUBSTITUTE**
ZWEIPHASIGER KERAMISCHER KNOCHENERSATZ
SUBSTITUT OSSEUX CÉRAMIQUE BI-PHASIQUE

(30) Priority: 23.03.2015 EP 15160388
(43) Date of publication of application: 31.01.2018
(73) Proprietor: Bone Support AB, 223 70 Lund (SE)
(72) Inventor: LIDGREN, Lars, 222 21 Lund (SE)
(74) Representative: Guardian IP Consulting I/S
(86) International application number: PCT/EP2016/056034
(87) International publication number: WO 2016/150876

(56) References cited:
- EP-A1- 2 353 619
- WO-A1-02/080933
- WO-A1-2012/094708

## Description

### FIELD OF INVENTION

The invention relates to synthetic bone grafts and their use in bone regeneration. More particular, the invention shows how a biphasic ceramic bone substitute can act as a carrier of both a bone active protein that can induce and/or stimulate bone growth, e.g. bone morphogenic proteins (BMP), and an anti-catabolic drug, e.g. a bisphosphonate, thereby being inductive and useful in improved active bone regeneration.

### BACKGROUND OF THE INVENTION

Fracture healing and bone remodeling can be seen as a form of tissue regeneration, and living bone is subject to constant remodeling with a complete turnover of bone mass in adults every 4-20 years. Bone remodeling is a cycle involving the 4 phases: activation, resorption, reversal and formation. Activation is probably started by the death or reformation of osteoclasts around a fracture or bone malignancy which recruit and induce new osteoclasts to start resorption of dead bone. After some time, resorption slows down and osteoblasts are recruited and activated in the reversal phase. Activated osteoblasts adhere to the surface after resorption of dead bone and start to produce new bone matrix-osteoid tissue, followed by a mineralization of the matrix. The action of the different bone cell types and their activators and inhibitors is balanced in a sensitive way normally leading to replacement of dead or fractured bone over time.

Restoration of serious bone defects such as loss of bone due to, i.a. trauma, eradication of infection, resection of tumor lesions, nonunion surgery and in primary or revision arthroplasties bone healing is often supported by surgical intervention where new bone formation is supported and accelerated, for example by use of bone grafts.

Autologous bone grafts are the ideal choice, because living cells and proteins within the graft is capable of inducing osteogenesis, i.e. de novo synthesis of bone. However, a limited supply and the risk of donor site morbidity after harvesting fresh bone have led to the use of bone allografts instead (for example bone from femoral heads resected at primary arthroplasty). Bone allografts, i.e. dead bone from subjects of the same species, which function as an osteoconductive scaffold are limited in supply and furthermore present a potential risk of inducing blood-born diseases, and even prohibited in some ethnical groups.

In several animal studies using cancellous bone grafts, the speed of remodeling and the volume of remodeled graft/substitute were found to be increased by bone morphogenic proteins (BMP), but most of the newly formed bone formed by BMP driven osteoinduction was resorbed almost as fast as formed. The reason for the resorption of the newly formed bone appears to be BMP activation of the Rank ligand system with enhanced recruitment of osteoclasts leading to premature catabolism. In clinical studies, the premature catabolism has led to loss of fixation in fractures, premature allograft resorption and failure and loosening in hip revision arthroplasty (Nicole Y.C. Yu, Aron Schindler, Magnus Tagil, Andrew J. Ruys, David G. Little. Frontiers in Bioscience E4, 2647-2653, June 1, 2012).

Bisphosphonates is a group of anti-catabolic drugs that inhibit bone resorption and they are clinically used in prevention and treatment of i.a. osteoporosis and bone metastases. Intravenously or orally administered bisphosphonates target and bind to bone mineral and such systemic applied bisphosphonates thus mainly accumulate in areas of active bone remodeling. During osteoclastic resorption, bisphosphonates bound to bone mineral are released and internalized in the osteoclasts followed by apoptosis of these cells. In animal studies, it has been shown that bisphosphonates applied intravenously or locally may inhibit resorption of newly formed bone induced by autografts or a combination of allografts and BMP (Yu et al. *ibis*). Toshihiko Nishisho et al. have disclosed a local administration of zoledronic acid together with artificial bone (hydroxyapatite or β-tricalcium) in the treatment of giant cell tumor of bone (Orthopedics Vol. 38, Issue 1: e25-e30 (2015) .

During the last decade, large bone defects have been treated with artificial grafts such as biomaterials that act as osteoconductive bone substitutes (Oryan A, Alidadi S, Moshiri A, Maffulli N. Bone regenerative medicine: classic options, novel strategies, and future directions. Journal of orthopaedic surgery and research. 2014; 9:18). These materials are polymeric, ceramic or of composite nature (Habibovic P, de Groot K. Osteoinductive biomaterials-properties and relevance in bone repair. Journal of tissue engineering and regenerative medicine. 2007; 1:25-32).

Other studies in bone tissue engineering involve incorporation of bone active proteins like bone morphogenic proteins (BMPs) and anti-catabolic drugs like bisphosphonates in porous polymers, sugar based high viscosity carriers or collagen. WO 2012/094708 discloses incorporation of BMP alone or combined with zoledronic acid (ZA) in biodegradable or biocompatible polymers. Hydroxyapatite may be doped with ZA and incorporated into the polymer when formed. WO 2014/032099 discloses compositions comprising a sugar based high viscosity carrier, BMP, bisphosphonate and optionally hydroxyapatite. Murphy CM, Schindeler A, Gleeson JP, Yu NYC, Cantrill LC, Mikulec K, et al. Acta Biomaterialia. 2014; 10:2250-8, discloses a collagen-hydroxyapatite scaffold which allows binding and co-delivery of recombinant BMPs and bisphosphonates. Studies involving a combination of BMP and bisphosphonates with carriers such as allografts or porous polymers have shown more or less synergistic results between BMP and the bisphosphonate. The polymers are pre-made standard products produced prior to insertion in a patient and therefore not easily adaptable for effectively use in filling individual bone voids for an efficient regeneration of bone without leaving empty spaces, which increases the risk of infection. The polymers are neither osteoconductive nor osteoinductive per se and therefore do not take active part in bone regeneration.

Injectable biphasic ceramic bone substitutes with the capability of being hardened in vivo to act as a synthetic graft, comprising a resorbable calcium sulphate hemihydrate component and a stable calcium phosphate component, such as for example hydroxyapatite, have been developed over the last years, for example by the Swedish company Bone Support AB (see: EP 1301219, EP 1465678, EP 1601387, EP 1829565, WO 2011/098438; WO 2014/128217). These publications suggest that the bone substitutes may contain an additive taken from a long list of biologically active agents. The list includes among others antibiotics, bone active proteins like bone morphogenic proteins (BMPs) and bisphosphonate. However, only antibiotics have so far been included in commercial bone substitutes. Bone Support AB has the three biphasic ceramic products on the market: CERAMENT™IBONE VOID FILLER, CERAMENT™ISPINE SUPPORT and CERAMENT™IG (CERAMENT™ with gentamicin), and a fourth product, CERAMENT™IV (CERAMENT™ with vancomycin) is now CE approved and ready for launch.

EP 2 353 619 A1 describes a bone cement composition comprising calcium sulfate hemihydrate powder, calcium phosphate powder, an accelerator for the hardening of the calcium sulfate hemihydrate, optionally an accelerator for the hardening of the calcium phosphate component, and a bioactive agent. The bioactive agent preferably induces, stimulates and/or accelerates bone formation.

Despite the many promising results in the use of more or less osteoconductive bone substitutes, an osteoinductive bone substitutes that could speed up the bone regeneration would be highly desirable.

### SUMMARY OF THE INVENTION

The scope of this invention is defined by the claims. Any references in the description to methods of treatment refer to the compounds, pharmaceutical compositions and medicaments of the present invention for use in a method for treatment of the human (or animal) body by therapy.

In the present invention, bone active protein(s) and anti-catabolic agent(s) are delivered together in an improved bone substitute to the bone defects by an osteoconductive carrier composed of a biphasic cement/ceramic material comprising at least one phase containing an anabolic agent that provides an initial microporosity and mechanical stability, and is resorbed in vivo, and at least one other phase that is stable and only slowly remodeled in vivo and preferably has as high affinity for an anti-catabolic agent.

The carrier material by itself is mainly osteoconductive, but the microporous and fast resorbable phase provides a controlled delivery of various added therapeutic agents and increases the macro porosity of the material allowing a fast ingrowth of new bone cells induced by bone growth factors, while the stable phase with its closely bound anti-catabolic agent is very slowly remodeled by new bone cells intruding through the porous material, whereby the anti-catabolic agent is slowly released over time and thus provides an ongoing local balancing of fast bone growth and resorption of new bone for the benefit of a formation of more dense and strong new bone.

The biphasic ceramic bone substitute according to the present invention, i.e. the ceramic material in a set state, comprises a) a calcium sulphate phase; b) a calcium phosphate phase; c) at least one bone active protein; and d) at least one anti-catabolic agent. The at least one bone active protein is present in the fast resorbable calcium sulphate phase and the at least one anti-catabolic agent is present in the stable calcium phosphate phase. The anti-catabolic agent is selected from bisphosphonic acids and bisphosphonates, i.e. an agent that inhibits bone resorption and has an affinity for the calcium phosphate.

The calcium sulphate phase preferably consists essentially of calcium sulphate dihydrate (CSD), also known as Gypsum. Needle-shaped calcium sulphate dihydrate particles are formed when calcium sulphate hemihydrate (CSH), also known as plaster of Paris, is reacted with water and the resulting CSD needles interlock to create a solid CSD matrix with a microporosity of about 20-40%. CSD is relative soluble in water and body fluids and therefore relatively quickly dissolved and fully resorbed in the body (within 6-12 weeks). Until being dissolved, the calcium sulphate phase provides a desirable mechanical strength to the bone support, which is often crucial for stability of the artificial graft after being implanted in the patient and for the hydroxyapatite particles not to migrate. In the process of CSD being dissolved and resorbed, the micropores are gradually enlarged in the bone substitute to form a matrix allowing stem cells (e.g. mesenchymal progenitor cells), activated osteoblasts, extracellular matrix (ECM) proteins and other bone cells to migrate from the lining between bone, bone marrow and the bone substitute deeper into the bone substitute, where new bone formation and remodeling can take place. The properties of calcium sulphate phase thus allow a progressive ingrowth of bone cells in the ceramic bone substitute while maintaining mechanical stability until newly formed bone secure mechanical stability.

In an embodiment of the present invention, the solid CSD is formed in a setting process, where calcium sulphate hemihydrate powder is mixed with water. Often it is necessary to add an accelerator for the process to occur within a desired and controllable time. If the substitute is to be injected or otherwise applied in liquid form (e.g. as a paste), for example in an in vivo treatment, a convenient setting time is between 10 and 30 minutes. As accelerant may be used calcium sulphate dihydrate or saline (NaCl solution).

The bone active proteins, and other bioactive agents if present (e.g. antibiotics), are preferably placed in and thus released from the calcium sulphate phase through the micropores of the calcium sulphate phase upon contact with body fluids. The fast release of the active agents after implantation in the patient leads to an initial high local concentration of bone active proteins and optionally other bone active factors in the bone substitute matrix and vicinity shortly after implantation, resulting in a strong initial stimulation of bone cell activation and growth.

In one embodiment of the invention, the bone active proteins and optionally any other bioactive agents are pre-mixed with the CSH powder prior to mixing with the calcium phosphate and liquid. In another embodiment, the bone active proteins and optionally any other bioactive agents are pre-mixed with the liquid before being mixed with the calcium sulphate and calcium phosphate. In a further embodiment the bone active proteins and optionally any other bioactive agents are pre-mixed with the calcium phosphate prior to mixing with the calcium sulphate and liquid. In yet another embodiment the bone active proteins and optionally any other bioactive agents are mixed with the paste right after mixing the calcium sulphate powder and the calcium phosphate powder with the liquid in a process known as "delayed mixing" (see WO 2011/098438). The latter may be relevant if the bone active proteins and optionally any other bioactive agents are disturbing the setting or setting time, for example if the setting time becomes too long for use in surgery of a patient. In any mixing event, the bone active proteins and optionally any other bioactive agents will end up in the calcium sulphate phase in the biphasic ceramic bone substitute of the present invention.

The calcium sulphate phase of the biphasic ceramic bone substitute of the present invention provides a unique carrier and delivery matrix for the bone active proteins, both allowing a controlled but relatively fast release of the bone active proteins and at the same time creating a beneficial porosity for fast bone cell ingrowth together with initial mechanical stability.

The calcium phosphate phase preferably consists essentially of calcium phosphate ceramics selected from the group consisting of α-tricalcium phosphate, hydroxyapatite, tetracalcium phosphate and β-tricalcium phosphate (see EP 1 301 219). A mixture of different calcium phosphate ceramics may be applied if desired. The calcium phosphate phase consists of amorphous and/or crystalline calcium phosphate particles. The particle size is preferably less than 200 µm, such as less than 100 µm, less than 50 µm, less than 35, less than 20 µm or less than 10 µm. (preferable between 0.1 and 50 µm). In one embodiment, the calcium phosphate is provided as calcium phosphate particles (e.g. sintered hydroxyapatite particles) to be mixed with the calcium sulphate powder and water for the calcium sulphate to set, whereby the calcium phosphate particles becomes embedding in the calcium sulphate phase after setting. In another embodiment, the calcium phosphate is provided as a hardenable calcium phosphate powder prepared for a setting reaction to form calcium phosphate cement upon mixing with water (see EP 1 301 219). The setting reaction of calcium phosphate may be accelerated by particulate calcium phosphate or a phosphate salt, for example disodium hydrogen phosphate (Na₂HPO₄).

In one particular embodiment of the present invention, the calcium phosphate phase consists essentially of hydroxyapatite particles. The hydroxyapatite particles may be in an amorphous or crystalline state. In a preferred embodiment the calcium phosphate phase consists essential of sintered crystalline hydroxyapatite particles. In one embodiment, the sintered crystalline hydroxyapatite particles are prepared in accordance with the method disclosed in WO 2014/128217, where sintered crystalline hydroxyapatite particles are inactivated by heating leading to improved setting properties of the calcium sulphate phase in a biphasic ceramic composition comprising crystalline hydroxyapatite and calcium sulphate. This is preferable when the composition comprises additional agents such as antibiotics.

In one embodiment of the present invention, the bone active proteins useful in the biphasic ceramic bone substitute of the invention are anabolic factors active in bone formation, i.e. preferably bone growth proteins selected from the group comprising bone morphogenic proteins (BMPs), insulin-like growth factors (IGFs), transforming growth factor-βs (TGFβs), parathyroid hormone (PTH), sclerostine, and the like. The bone active proteins may also be provided in the form of a composition comprising cell factory-derived bone active proteins, and ECM proteins (WO 2008/041909). Alternatively strontium as a bone growth factor may be used in addition to or as a substitute of the bone active proteins.

In a preferred embodiment, the bone active protein could be bone growth proteins selected from the long list of BMPs, but most preferably BMP-2 or BMP-7 or a combination thereof. BMPs may be isolated from donor cells (e.g. from a bone cell factory) or prepared recombinantly. For human patients recombinant human BMPs, such as rhBMP-2 or rhBMP-7 are preferably used. rhBMPs are commercially available or may be produced by known techniques. Bone active proteins may be provided as such and added to any of the powders, the aqueous liquid or the paste. Alternatively, the bone active proteins may be encapsulated in water-soluble and/or biodegradable synthetic polymeric microcapsules, bovine collagen particles, starch particles, dihydrate nidation particles, or the like before use. Encapsulated active additives have the advantage of being protected during storage and mixing in addition to the possibility of being prepared well in advance before use. The encapsulated active additives may be released before or in the paste or during dissolution and resorption of the calcium sulphate phase.

According to the present invention, the anti-catabolic agents useful in the biphasic ceramic bone substitute of the present invention are agents which inhibit bone resorption, and which are selected from bisphosphonic acids and bisphosphonates.

In a preferred embodiment the anti-catabolic agent is a bisphosphonate. The bisphosphonates have a strong affinity for bone minerals, i.e. calcium phosphates such as hydroxyapatite, and they can be divided into simple bisphosphonates (e.g. etidronate) and nitrogen-containing bisphosphonates (e.g. alendronate and zoledronate). The potency of the different bisphosphonates should be considered when selecting a bisphosphonate for use in the biphasic ceramic bone substitute according to the present invention. Alendronate is 10-100 times more potent than etidronate and zoledronate up to 10.000 times more potent than etidronate. Bisphosphonates target and bind to bone mineral due to their molecular structure and their ability to chelate calcium ions. Due to their strong affinity to minerals in bone, they accumulate in areas of active remodeling and minimally to other cell types and they practically remain bound until they are released during bone resorption where they are internalized in osteoclasts. However, as the bisphosphonates are toxic to osteoclasts, these go into apoptosis whereby the bone resorption is inhibited or sustained.

The calcium phosphate phase of the biphasic ceramic bone substitute of the present invention provides a unique carrier and delivery matrix for the anti-catabolic agents, preferably bisphosphonates, both allowing a controlled and slow release of the agents at the same rate as newly formed bone cells are created and differentiated into osteoclast, i.a. as a result of BMP activation, and at the same time forming a stable matrix which is very slowly resorbed (4-12 months) or incorporated into the newly formed bone after the calcium sulphate has been resorbed. Compared to some of the known polymeric carriers, the calcium phosphate phase will be resorbed or incorporated as a natural mineral over time, leaving no artificial polymer in the patient. The anti-catabolic agents (e.g. bisphosphonates) present in the biphasic ceramic bone substitute according to the present invention suppress premature resorption of newly formed bone by osteoclasts in and connected to the biphasic bone substitute at a pace following the ingrowth of new bone, because the anti-catabolic agents bound to the calcium phosphate particles in the matrix becomes exposed as a consequence of dissolution and resorption of the calcium sulphate phase. Newly formed bone cells thus meet the anti-catabolic agent when expanding into the matrix from the graft being inserted in the bone defect until and beyond full mineralization of the newly formed bone. The suppression of premature resorption leads to a more dense formation and mineralization of new bone as seen in an animal muscle model and will help cure patients with bone defects in a better and faster time than previously seen. The whole or part of the calcium phosphate may be pretreated with bisphosphonate and thereby bound for an optimal balance and bone ingrowth starting immediately but extending over a longer period of 12-24 months

While known polymeric carriers may only comprise a small amount of hydroxyapatite (2% (w/v) in WO 2012/094708 and 1-5% (w/v) in WO 2014/032099), the biphasic ceramic carrier of the present invention may comprise up to about 95% (w/w) calcium phosphate (e.g. hydroxyapatite) (about 40% hydroxyapatite in the Cerament™ products on the market), thus allowing the bisphosphonates to be dispersed at a much higher density in the carrier of the present invention. The higher density secures a more effective local inhibition of bone cell resorption by intruding osteoclasts, leaving the scene to the osteoblasts. Furthermore, while only some polymers provide a mechanical support and none of the polymers are ideal as bone grafts as they are not very osteoconductive, the biphasic ceramic bone substitute carrier (e.g. a Cerament™ product) used in the present invention is microporous, mechanical supportive, osteoconductive and osteoinductive. Porous polymers which provide mechanical support, e.g. poly((lactic-co-glycolic) acid) (see WO 2012/094708), require solvents and/or temperatures or has an exothermic polymerization process that make them unsuited for in vivo polymerization and thus insertion by injection. Injectable polymers, e.g. sugar based high viscosity polymers (see WO 2014/032099) provide low porosity and no mechanical support.

The anti-catabolic agent may be provided as a powder or solution and/or may be encapsulated in water-soluble and/or biodegradable synthetic polymeric microcapsules, bovine collagen particles, starch particles, dihydrate nidation particles, or the like. Encapsulated active additives have the advantage of being protected during storage and mixing in addition to the possibility of being prepared and stored well in advance before use. When added as an encapsulated ingredient, bisphosphonates are released from their encapsulation and bound to the neighboring calcium phosphate particles, such as for example when the encapsulations are contacted with water, for example when preparing the paste or in vivo when body fluids get access to the capsulations. The anti-catabolic agent and the bone active protein may be provided in the same or different encapsulations.

In an embodiment of the present invention, the biphasic ceramic bone substitute further comprises one or more additional bioactive agent selected from antibiotics (including antifungal drugs), bone healing promotors, chemotherapeutics, cytostatics, vitamins, hormones, bone marrow aspirate, platelet rich plasma and demineralized bone. In a preferred embodiment, the biphasic ceramic bone substitute comprises one or more antibiotics (e.g. gentamicin and/or vancomycin). The additional bioactive agent(s) may be mixed with the calcium sulphate powder, the calcium phosphate powder/particles or with the liquid, or may be mixed with the paste comprising the calcium sulphate powder, the calcium phosphate powder/particles and the liquid in a delayed mixing process as has described above. Also the additional bioactive agents may be encapsulated in water-soluble and/or biodegradable synthetic polymeric microcapsules, bovine collagen particles, starch particles, dihydrate nidation particles, or the like. The additional bioactive agent(s) may be provided in the same or different encapsulations optionally together with the anti-catabolic agent and/or the bone active protein and released before or in the paste or by in vivo contact with body fluids.

In yet another embodiment of the present invention, the biphasic ceramic bone substitute also comprises an X-ray contrast agent selected from water soluble non-ionic X-ray contrast agents (e.g. iohexol) and/or biodegradable X-ray contrast agents. The X-ray contrast agent may be mixed with the calcium sulphate powder, the calcium phosphate powder, other additives or with the liquid, or may be mixed with the paste comprising the calcium sulphate powder, the calcium phosphate powder and the liquid in a delayed mixing process as described above. X-ray contrast agents may also be encapsulated in water-soluble and/or biodegradable synthetic polymeric microcapsules, bovine collagen particles, starch particles, dihydrate nidation particles, or the like, if desirable. The X-ray agent(s) may be provided in the same or different encapsulations optionally with the anti-catabolic agent and/or the bone active protein and/or other additives and released before or in the paste. A premixed X-ray solution comprising iodine (iohexol) for enhancing x-ray capacity ready for mixing with ceramic powders is available from BONESUPPORT AB under the trade name CERAMENT™IC-TRU.

In a specific embodiment of the present invention biphasic ceramic materials from BONESUPPORT AB, such as CERAMENT™I BONE VOID FILLER, CERAMENT™I SPINE SUPPORTCERAMENT™IG and CERAMENT™IV may act as a carrier for bone active agent(s) like bone morphogenic proteins (BMPs) and anti-catabolic agent(s) like bisphosphonates. Table 1 shows the content of commercial Cerament™ products. It has been demonstrated in the present invention that the hydroxyapatite present in the Cerament™ products can act osteoinductive on stem cells and that it has a low immunogenicity.

**Table 1 Specification of composition of CERAMENT™ products.**

| **Product name** | **CERAMENT™I SPINE SUPPORT** | **CERAMENT™I BONE VOID FILLER** | **CERAMENT™IG** | **CERAMENT™IV** |
|---|---|---|---|---|
| **Ceramic powder Composition pre filled in a combined mixing and injection device (CERAMENT™ICMI*** | 59.6 % CSH | 59.6 % CSH | 59.6 % CSH | 59.6 % CSH |
| | 0.4 % CSD | 0.4 % CSD | 0.4 % CSD | 0.4 % CSD |
| | 40.0 % HA | 40.0 % HA | 40.0 % HA | 40.0 % HA |
| **Type of liquid phase** | Iohexol solution (300 mg I/mL) CERAMENT™IC-TRU | Iohexol solution (180 mg I/mL) CERAMENT™IC-TRU | Saline (9 mg NaCl/mL) CERAMENT™IMIXING LIQUID | Iohexol solution (180 mg I/mL) CERAMENT™IC-TRU |
| **L/P ratio** | 0.50 mL/g | 0.43 mL/g | 0.43 mL/g | 0.43 mL/g |
| **Type of antibiotic** | - | - | Gentamicin sulfate | Vancomycin hydrochloride |
| **Concentration of antibiotic** | - | - | 2.2 wt-% Gentamicin sulfate** | 5.4 wt% Vancomycin** |
| | | | *(17.5 mg Gentamicin*/*mL paste)* | *(66 mg Vancomycin*/*mL paste )* |

| | | | | |
|---|---|---|---|---|
| * CSH=Calcium sulfate hemihydrate; HA= Hydroxyapatite; CSD=calcium sulfate dihydrate; **concentration based on ceramic powder | | | | |

For the purpose of the present text, "Cerament™ products" means one or more of the powder compositions present in CERAMENT™I BONE VOID FILLER, CERAMENT™I SPINE SUPPORT, CERAMENT™IG and CERAMENT™IV and denoted CERAMENT™IBVF or CERAMENT™BVF or Cerament™BVF; CERAMENT™SS or Cerament™SS; CERAMENT™G or Cerament™G; and CERAMENT™V or Cerament™V, respectively. Pastes and set solid bone support produced from these powder compositions by mixing with a liquid may be mentioned by the same names throughout the text. The state and content of a "Cerament™ product" will be clear from the context.

It has been shown that high initial release of bone active proteins (e.g. BMP-2) and a sustained release of bisphosphonates (e.g. ZA) from Cerament™ products makes it an excellent carrier platform. The initial high release of bone active proteins as seen in-vitro is attributed to the biphasic material with resorbable calcium sulphate and initial microporosity. The increased availability of such proteins to the inducible cells leads to early onset of differentiation that in turn can provide accelerated bone growth. In contrast, the sustained but low release of bisphosphonates from the carrier platform seen in-vitro is caused by strong binding of bisphosphonates to the surface of the calcium phosphate (e.g. hydroxyapatite) particles. The sustained release and exposure of bisphosphonates to new bone cells inhibits premature resorption of newly formed bone and thus allows maturation and mineralization of new bone cells to result in a fast formation of strong remodeled bone.

In one embodiment of the present invention, the biphasic ceramic bone substitute may be prepared as beads in mold(s) and/or sculptured in any desired form prior to implantation in a patient. Setting time for the material may not be critical in preset beads or sculptured preparations. In another embodiment of the present invention, the biphasic ceramic bone substitute is the result of an in vivo setting process where a biphasic ceramic bone substitute paste according to the present invention is injected or otherwise placed at the site of the bone defect in the patient. In such an in vivo setting process, the setting time is often critical. The right combination of setting components, additives and accelerators is prerequisite for an optimal, consistent and reliable setting of the bone substitute. The paste may be prepared immediately prior to use by mixing the dry powders with an aqueous liquid, which may comprise some or all of the water soluble additives. Some or all of the additives may be premixed with one or different dry powders before mixing with the aqueous liquid. Some or all of the additives may be added to and mixed with the paste before being used and before setting. Some or all of the additives may in an encapsulated form for later release as described above.

In a further embodiment of the present invention, the powders and additives may be provided in a kit ready for mixing, where the different powders and additives are provided individually or pre-mixed in any desirable way or combination in different containers. The kit may also comprise an aqueous liquid for preparing the paste and the liquid may contain one or more of the additives.

Additionally, the kit may contain instructions for mixing and use and/or mixing and injecting devices, including a syringe, such as for example disclosed in WO 2005/122971.

The biphasic ceramic bone substitute according to the present invention may be used in the treatment of most bone defects where surgical intervention and filling of voids are needed and/or beneficial, such as loss of bone due to i.a. trauma, debriding of infected areas, resection of pathological lesions (e.g. bone cancer), nonunion surgery and in primary or revision arthroplasties. Bones to be treated include, but are not limited to, the spinal cord, bones of the hands, fingers, arms, feet, toes, lower or upper legs, knee, hip, ankle, elbow, wrist, shoulders, skull, jaw and teeth of any animal or a human.

### DRAWINGS

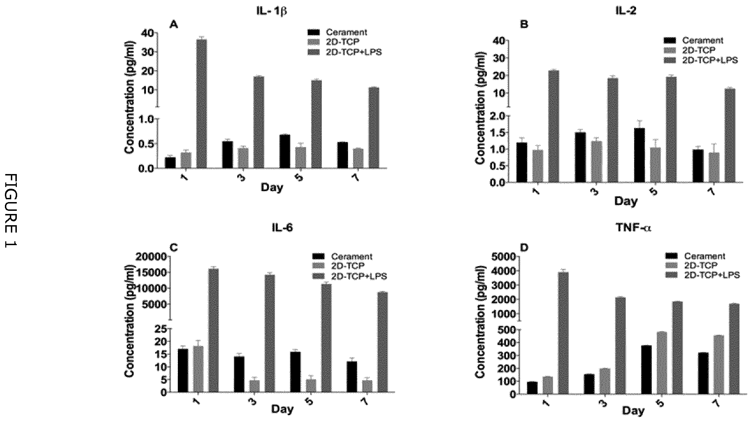

Figure 1: in-vitro immunogenicity analysis of Cerament™. RAW 264.7 cells were seeded on Cerament™BVF and the release of various pro inflammatory cytokines IL-1β (Panel A), IL-2 (Panel B), IL-6 (Panel C) and TNF-a (Panel D) was analyzed and compared to LPS as an immunogen.

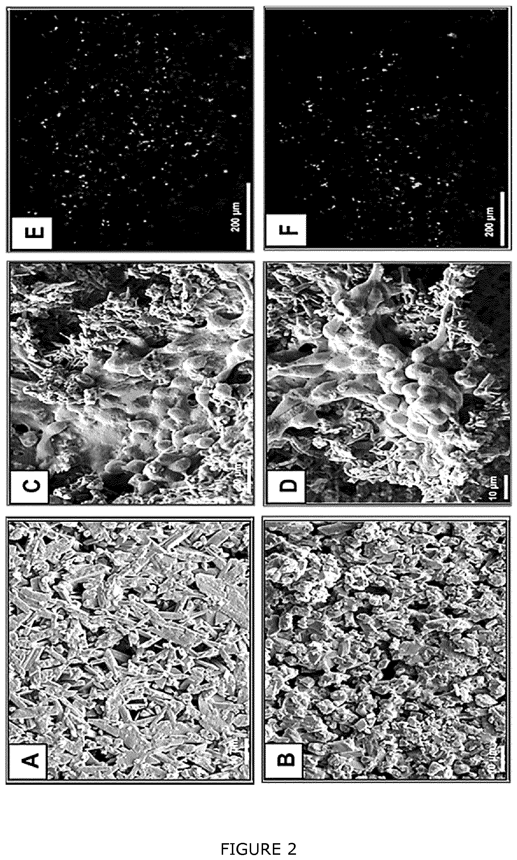

Figure 2: Cell-material interactions via electron microscopy. Panels A and B represent Cerament™BVF and Cerament™G, respectively. Panels C and D represent attachment of C2C12 cells on the surface of both materials (BVF/G) while panels E and F represent nuclear staining of C2C12 cells (using DAPI) to show homogenous distribution of cells all across the surface of the two materials (BVF/G).

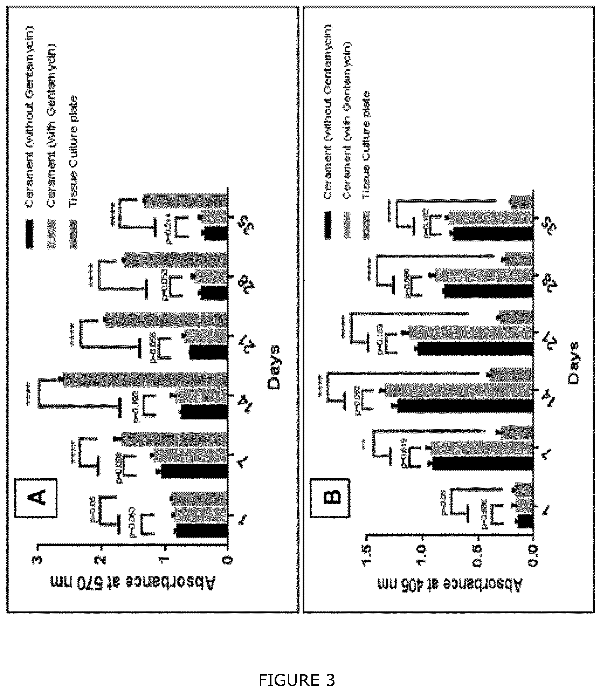

Figure 3: Cell culture study on Cerament™BVF materials using C2C12 cells via MTT and ALP analysis. Panel A represents the proliferation pattern of C2C12 muscle myoblasts on Cerament™BVF and Cerament™ G biomaterials over a period of 5-weeks post-seeding. Cellular proliferation was assessed via MTT assay with 2D-polystyrene plates as a control for proliferation. Panel B represents alkaline phosphatase assay showing the level of ALP activity of C2C12 muscle myoblasts seeded on Cerament™BVF and Cerament™G compared with tissue culture plate over a period of 35 days.

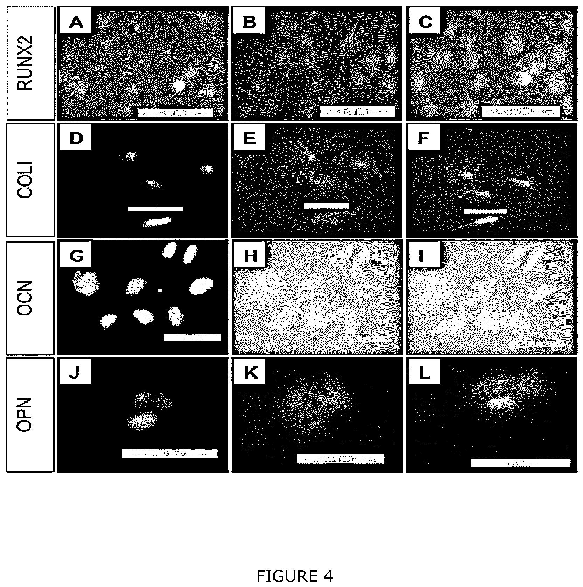

Figure 4: Immunocytochemical and RT-PCR analysis of C2C12 muscle myoblasts seeded on Cerament™. C2C12 cells seeded on Cerament™BVF were analyzed using immunocytochemistry to visualize osteogenic differentiation. Cells were stained after a period of 7 and 21- days post seeding. Cells stained positive for osteogenic markers like RunX-2 (A-C) at day 7, Col I (D-F), OCN (G-I) and OPN (J-L), respectively after 21-days post seeding. Images in the left panel (A, D, G and J) indicates nuclear staining using DAPI while images in middle panels (B, E, H and K) indicate antibody based detection of respective target proteins while right panels (C, F, I and L) depict respective merged images.

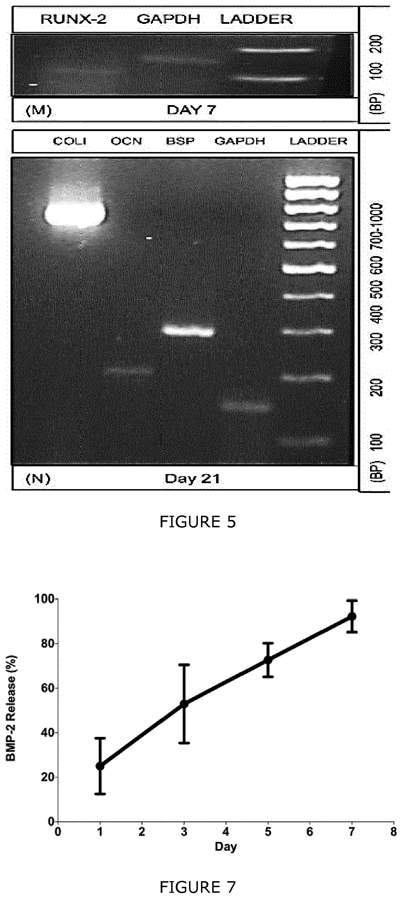

Figure 5: Early onset of osteogenic differentiation was confirmed by the presence of RunX-2 gene in C2C12 cells seeded on Cerament™BVF after 7-days (Panel M). Osteoblastic maturation (21 days post seeding) of muscle cells was confirmed by the presence of osteoblastic genes coding for Col I (Panel N, Lane 1), OCN (Panel N, Lane 2), BSP (Panel N, Lane 3), housekeeping gene GAPDH (Panel N, Lane 4) with control ladder in Panel N, lane 5.

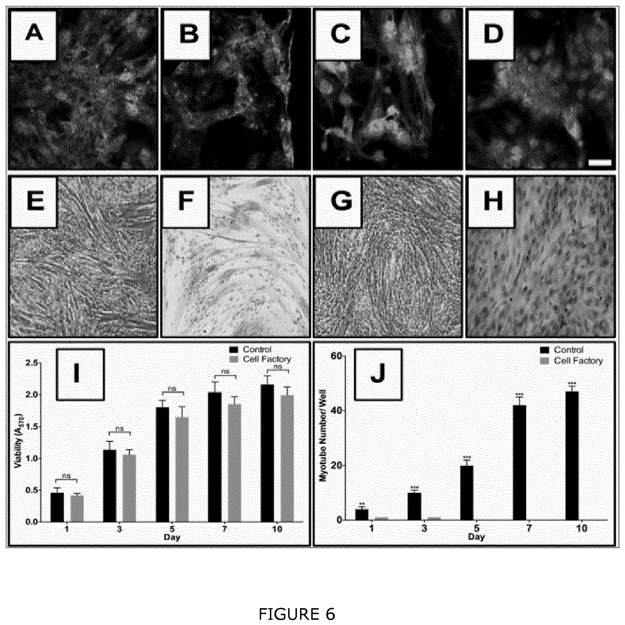

Figure 6: Morphological and phenotypical changes in skeletal muscle cells L6 after treatment with cell factory bone active proteins. Panels A-D show the expression of Col I, OCN, OPN and BSP, respectively 12-days post seeding in the experimental group. Cells stained positive for most prominent osteoblastic markers COLI (Panel A), OCN (Panel B), OPN (Panel C) and BSP (Panel D). Panels E&F indicate myotube formation in the control groups while cell factory treated group shows uni-nuclear morphology (Panels G&H). Panel I indicates cell proliferation in both groups while panel J shows myotube numbers for both groups.
Figure 7: In-vitro release profile of BMP-2 from Cerament™ discs.

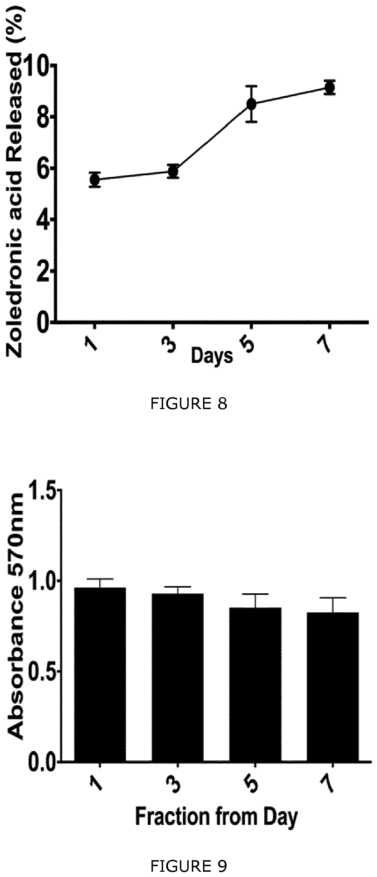

Figure 8: In-vitro release profile of ZA from Cerament™ discs over time.
Figure 9: Cytotoxicity induced by released ZA from Cerament™ discs on A549 tumor cells.

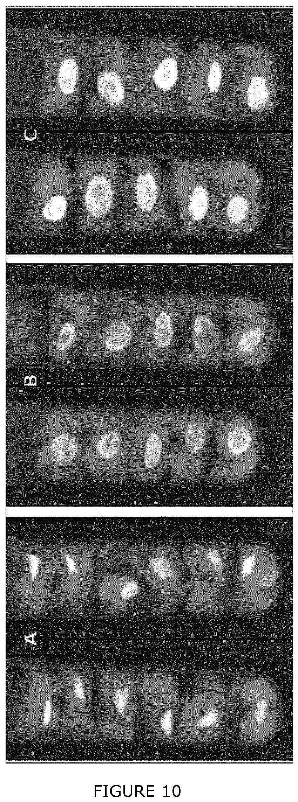

Figure 10: Radiographs showing Cerament™BVF discs implanted in the abdominal muscle pouch for 4-weeks. Panel A shows Cerament™BVF, B shows Cerament™BVF+rhBMP-2 and C shows Cerament™BVF+rhBMP-2+ZA.

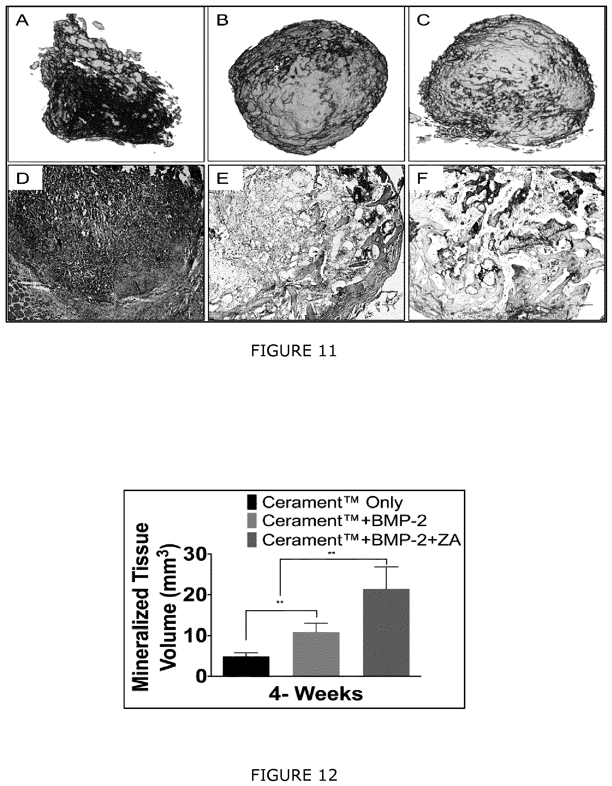

Figure 11: Micro-CT & Histology of Cerament™BVF discs implanted in the abdominal muscle pouch for 4-weeks. Panels A-C represent 3D micro-CT reconstructions of Cerament™BVF, Cerament™BVF+rhBMP-2 and Cerament™BVF+rhBMP-2+ZA, respectively. Panels D-F represents histology (H&E) of Cerament™BVF, Cerament™BVF+rhBMP-2 and Cerament™BVF+rhBMP-2+ZA, respectively after 4-weeks of in-vivo implantation.
Figure 12: Mineralized tissue volume in Cerament™BVF, Cerament™BVF+rhBMP-2 and Cerament™BVF+rhBMP-2+ZA, respectively after 4-weeks of in-vivo implantation.

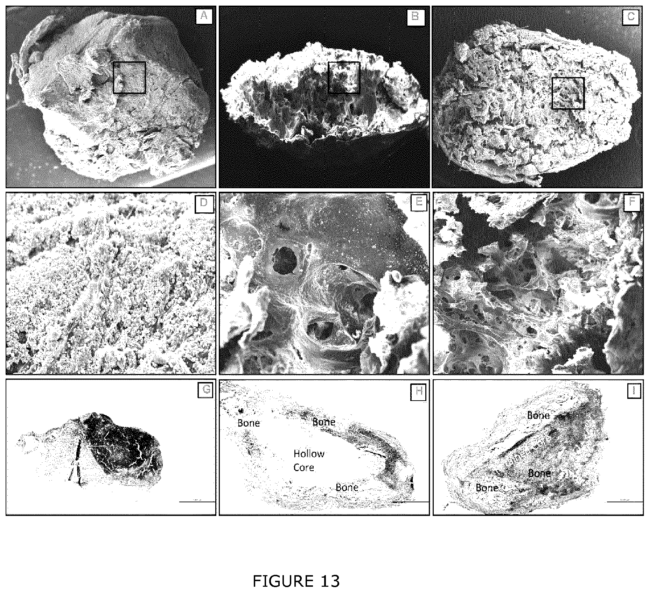

Figure 13: Micro-CT & Histology of Cerament™BVF discs implanted in the abdominal muscle pouch for 4-weeks. Panels from A-C (first row) represent 3D micro-CT reconstructions of Cerament™BVF, Cerament™BVF+rhBMP-2 and Cerament™BVF+rhBMP-2+ZA, respectively. Area taken for histology and EM indicated. The second row shows EM for Cerament™BVF with no bone cells (D); Cerament™BVF+rhBMP-2 with bone in the periphery only (E); and Cerament™BVF+rhBMP-2+ZA with trabecular bone bridging over the central part (F). The third row (G-I) shows the same as the second row above at a lower magnification.

### DETAILED DESCRIPTION OF THE INVENTION

The present invention concerns new biphasic ceramic bone substitute for use in the treatment of disorders of supportive tissue such as regeneration of bone defects, in particular serious bone defects where a graft is needed. The two phases in the ceramic bone substitute consists of a relatively fast resorbable calcium sulphate phase and a very slowly resorbable calcium phosphate phase. The biphasic ceramic bone substitute further comprises at least one bone active protein that served as an osteoinductive factor for regeneration of new bone, and at least one anti-catabolic agent that inhibits bone resorption. The combination of bone active proteins, specific inhibitors of bone resorption and a biphasic ceramic bone substitute carrier comprising a microporous and relatively fast resorbable phase and a very slow resorbable phase has proven to be surprisingly beneficial.

**The calcium sulphate** phase of the biphasic ceramic bone substitute essentially consists of calcium sulphate dihydrate that is formed in a setting process where calcium sulphate hemihydrate is reacted with water, whereby calcium sulphate dihydrate crystals are formed over time and interlock with each other to for a microporous matrix. The setting reaction may be accelerated by addition of 0.1-10, such as 0.2-5 weight% calcium sulphate dihydrate or a suitable salt, e.g. in the form of a solution, for example saline (NaCI-solution). During the setting process, calcium phosphate particles in the calcium phosphate phase (e.g. hydroxyapatite particles) are embedded in the voids of microporous calcium sulphate dihydrate matrix (the calcium sulphate phase). The calcium sulfate phase provides an initial mechanically solid property to the bone substitute. The microporosity and the relatively fast resorption of the calcium sulphate phase in the body liberates additive present in the calcium sulphate phase at an initial high rate and the artificial material is transformed into a very porous skeleton along with the resorption of calcium sulphate resulting in an increased access of body fluids and cells to the calcium phosphate particles in the calcium phosphate phase. This has shown to be highly beneficial in (fast) bone cell ingrowth. In an in-vitro assay it is shown that BMP-2 is released from solid Cerament™ bone support at a constant rate over a period of 7-days with nearly 90% of BMP-2 released after 7-days.

**The calcium phosphate** phase of the biphasic ceramic bone substitute essentially consists of calcium phosphate particles selected from the group consisting of α-tricalcium phosphate, hydroxyapatite, tetracalcium phosphate and β-tricalcium phosphate. The calcium phosphate component may be added as preset particles or added as hardenable precursors (powder) for a setting process within the biphasic material upon addition of water. Accelerators of such setting processes, e.g. particulate calcium phosphate particles and phosphate salts, are known in the art and may be added in the process. The calcium phosphate particles may be amorphous or crystalline in structure. A desired structure may be obtained by e.g. heat-treatment, re-crystallization and/or dissolution processes known in the art. EP 1 301 219, EP 1 465 678 and EP 1 601 387 disclose calcium phosphates, their preparation and their use in ceramic bone substitutes.

In a preferred embodiment of the invention, the calcium phosphate phase is essentially composed of hydroxyapatite, in particular crystalline hydroxyapatite particles. Anti-catabolic agents such as bisphosphonate have a strong affinity to calcium phosphates, such as hydroxyapatite, which constitutes the calcium phosphate phase in the commercially available Cerament™ products.

WO 2014/128217 discloses passivated crystalline hydroxyapatite particles and their use in ceramic bone substitutes. Crystalline hydroxyapatite powder is heated after being sintered and grinded or milled, which surprisingly leads to passivation (inactivation) of the crystalline hydroxyapatite particles that otherwise may interfere with the setting process of the calcium sulphate phase, especially when the bone substitute powder comprises additives such as an antibiotic agent. Passivated crystalline hydroxyapatite particles may advantageously be used in the present invention.

### Bone active proteins

Bone active proteins included as an additive in the biphasic ceramic bone substitute are preferably selected from bone growth proteins such as from the group comprising bone morphogenic proteins (BMPs), insulin-like growth factors (IGFs), transforming growth factor-βs (TGFβs), parathyroid hormone (PTH), sclerostine, and the like. Alternatively, one or more bone active proteins can be provides as a composition of cell factory derived bone active proteins and/or extracellular matrix proteins (ECM). More alternatively, strontium may be used in addition to or substitute the bone active proteins. In one embodiment, the bone active proteins are mixed with the calcium sulphate hemihydrate powder before mixing the sulphate hemihydrate and calcium phosphate powders. Alternatively, the bone active proteins are mixed with the mixed sulphate hemihydrate and calcium phosphate powder or the aqueous liquid or added to the paste. The bone active proteins may be provided as encapsulated in water-soluble and/or biodegradable polymer(s). In one embodiment the bone active protein is the bone growth protein, preferably a bone morphogenic protein (BMP). Preferably the BMP is BMP-2 or BMP-7. In a specific embodiment the BMP is a recombinant BMP, preferably recombinant human BMP, such as rhBMP-2 or rhBMP-7. BMP is used in a concentration of 0.2 to 500 µg/g dry powder, more preferably 1.0-250, or 2-200, or 5-1500, or 10-120 µg BMP/g dry powder. Other bone active proteins may be used in a similar or corresponding concentration or a concentration necessary for obtaining the desired effect.

The bone active proteins are incorporated into the bone substitute by addition to and mixing with either the bone substitute powder or the aqueous liquid. Alternatively, the bone active proteins may be added to the paste before casting. In a preferred embodiment, the bone active proteins are pre-mixed with the calcium sulphate powder before mixing with the calcium phosphate powder.

Bone active proteins may be provided as such and added to any of the powders, the aqueous liquid or the paste. Alternatively, the bone active proteins may be encapsulated in water-soluble and/or biodegradable synthetic polymeric microcapsules, bovine collagen particles, starch particles, dihydrate nidation particles, or the like before use.

### The anti-catabolic agent

One or more anti-catabolic agent(s) for inclusion in the biphasic ceramic bone substitute of the present invention are selected from bisphosphonates and bisphosphonic acids. Preferably the anti-catabolic agent is one or more bisphosphonates.

Bisphosphonates are divided into non-nitrogenous (or simple) bisphosphonates and N-containing bisphosphonates. The N-containing bisphosphonates are more potent than the simple bisphosphonates.

The simple bisphosphonates are metabolized in the cell to compounds that replace the terminal pyrophosphate moiety of ATP, forming a nonfunctional molecule that competes with adenosine triphosphate (ATP) in the cellular energy metabolism. The osteoclast initiates apoptosis and dies, leading to an overall decrease in the breakdown of bone. Examples of simple bisphosphonates are etidronate, clodronate and tiludronate. Clodronate and tiludronate are 10 times as potent as etidronate.

Nitrogenous bisphosphonates act on bone metabolism by binding and blocking the enzyme farnesyl diphosphate synthase (FPPS) in the HMG-CoA reductase pathway (also known as the mevalonate pathway). Examples of N-containing bisphosphonates are (potency relative to etidronate are given in parenthesis): pamidronate (100), neridronate (100), olpadronate (500), alendronate (500), ibandronate (1000), risedronate (2000) and zoledronate (10000).

The bisphosphonates may be added in solution to the calcium phosphate particles/powder (e.g. hydroxyapatite) where it strongly binds to calcium phosphate prior to mixing with the calcium sulphate powder. The amount/concentration of bisphosphonates necessary for obtaining a desired effect depends, i.a. on the potency of the bisphosphonate selected. The concentration of bisphosphonate in "doped" calcium phosphate particles may be controlled by selecting the bisphosphonate concentration in the solution and/or the time the particles are placed in the bisphosphonate solution. Alternatively, the amount/concentration of bisphosphonates in the powders and the paste may be controlled by using a mixture of calcium phosphate particles (e.g. hydroxyapatite) doped with a known (high) amount/concentration of bisphosphonate and un-doped calcium phosphate particles in a desired ratio.

In a preferred embodiment, the selected bisphosphonate is zoledronate/zoledronic acid (ZA).

ZA is used in a concentration of 0.2 to 500 µg/g dry powder, more preferably 1-300 µg/g, or 10-200 µg/g, or 10-120 µg/g. Other bisphosphonates may be used in a similar or corresponding concentration or a concentration necessary for obtaining the desired effect. The dosages of BMP used in local application in accordance with the present invention may be as low as 20% of what is needed in systemic infusion or even lower. Too high dosages of bisphosphonates (e.g. ZA) are toxic and will alone lead to an inflammatory reaction and also not only kill osteoclast but also impair the osteoblasts. In addition high dosages of BMP alone can lead to a strong reaction and a too extensive bone formation.

Bone active proteins may be provided as a powder or a solution and added to any of the powders, the aqueous liquid or the paste. Alternatively, the bone active proteins may be encapsulated in water-soluble and/or biodegradable synthetic polymeric microcapsules, bovine collagen particles, starch particles, dihydrate nidation particles, or the like before use.

Discs for use in an in-vitro ZA release assay were prepared by mixing ZA with a ceramic powder (Cerament™BVF), a liquid and cast in molds. Saline was added to the discs and at different time points, a sample of the medium was harvested and analysis. The release of ZA from each Cerament™BVF discs can be calculated in the harvested supernatants by adding lung cancer cells (cell line A549) wherein ZA is known to induce apoptosis. After a period of 7-days, the amount of ZA released from Cerament™BVF was about 10% of the total ZA loaded.

### Additional bioactive agents

The biphasic ceramic bone substitute according to the invention may also comprise at least one further bioactive agent. Such bioactive agents are selected from antibiotics (including antifungal drugs), bone healing promotors, chemotherapeutics, cytostatics, vitamins, hormones, bone marrow aspirate, platelet rich plasma and demineralized bone.

An antibiotic agent is preferably selected from gentamicin, vancomycin, tobramycin, cefazolin, rifampicin, clindamycin and the antifungal drug is preferably selected from the group comprising nystatin, griseofulvin, amphotericin B, ketoconazole and miconazole. The ceramic powder product CERAMENT™IG marketed for use in bone substitution comprises gentamicin. A new ceramic powder product, CERAMENT™IV, for use in bone substitution comprises vancomycin.

Concentrations in additional bioactive agent depend on the agent and desired effect. For the antibiotics gentamicin and vancomycin, they are used in an amount of 0.5 to 10 weight% of the ceramic powder, preferably between 1 and 6 weight%.

If it is desired to have further bioactive agents in the bone substitute (in addition to bone active protein and anti-catabolic agent), these may be added to and comprised in the powder or in the aqueous liquid. Alternatively, one or more of additional bioactive agents may be added to the paste before setting.

Additional bioactive agents may be provided as such and added to any of the powders, the aqueous liquid or the paste. Alternatively, the bioactive agents may be encapsulated in water-soluble and/or biodegradable synthetic polymeric microcapsules, bovine collagen particles, starch particles, dihydrate nidation particles, or the like before use.

### X-rav contrast agents

In implantation situation, it is often important for the surgeon to be able to follow the placement of the biphasic ceramic bone substitute in the patient during and after the surgery. It may also be helpful to be able to follow ingrowth of new bone or failures that need to be corrected. In one embodiment of the present invention an X-ray contrast agent selected from water soluble non-ionic X-ray contrast agents and/or biodegradable X-ray contrast agents may be incorporated into the bone substitute. EP 1 465 678 and WO 2014/128217 disclose incorporation of x-ray contrast agents into ceramic bone support. The X-ray contrast agents may be added to constitute 1-25 weight% of the total powder ingredients, preferable 10-25 weight%.

In a preferred embodiment the water soluble non-ionic X-ray contrast agent is selected from iohexol, iodixanol, ioversol, iopamidol, iotrolane, metrizamid, iodecimol, ioglucol, ioglucamide, ioglunide, iogulamide, iomeprol, iopentol, iopromide, iosarcol, iosimide, iotusal, ioxilane, iofrotal, and iodecol. In another embodiment, biodegradable X-ray contrast agents which may provide additional pores may be used.

The X-ray contrast agent may be provided as such and added to any of the powders, the aqueous liquid or the paste. Alternatively, the X-ray contrast agent may be encapsulated in water-soluble and/or biodegradable synthetic polymeric microcapsules, bovine collagen particles, starch particles, dihydrate nidation particles, or the like before use.

### Ceramic bone substitute powder

In a further embodiment, the present invention concerns a hardenable ceramic bone substitute powder comprising:
a. calcium sulphate hemihydrate powder;
b. calcium phosphate powder, where the calcium phosphate is selected from one or more of the group consisting of α-tricalcium phosphate, hydroxyapatite, tetracalcium phosphate and β-tricalcium phosphate,;
c. a bone active protein;
d. an anti-catabolic agent which inhibits bone resorption, selected from bisphosphonic acids and bisphosphonates;
e. optionally an accelerator for setting of calcium sulphate preferably selected from calcium sulphate dihydrate and a salt (e.g. NaCI); and
f. optionally an accelerator for setting of calcium phosphate preferably particulate calcium phosphate and/or a phosphate salts (e.g. Na₂HPO₄).

"Hardenable ceramic bone substitute powder" means that calcium sulphate hemihydrate powder and optionally the calcium phosphate powder will set as a solid material after contact with a liquid.

The biphasic ceramic bone substitute powder (basis powder with or without additives) according to the present invention comprises a calcium sulphate hemihydrate to calcium phosphate ratio (w/w) from 5:95 to 95:5, from 10:90 to 90:10, from 20:80 to 80:20, from 30:70 to 70:30, or from 40:60 to 60:40. Cerament™s on the market comprises 59.6 weight% calcium sulphate hemihydrate and 40 weight% hydroxyapatite.

In a preferred embodiment, the calcium phosphate powder is a preset hydroxyapatite powder, preferable comprised of amorphous and/or crystalline hydroxyapatite particles.

Calcium phosphate particles (e.g. crystalline hydroxyapatite) for use as preset calcium phosphate powder have a particle size of D(v,0.99) <200 µm, preferably <100 µm and more preferably <50 µm, such as less than 35 µm. The specific surface area of the powder should preferable be below 20 m²/g, and more preferably below 10 m²/g, when measured according to the BET (Brunauer, Emmett and Teller) method, which is a method for the determination of the total surface area of a powder expressed in units of area per mass of sample (m²/g) by measurement of the volume of gas (usually N₂) adsorbed on the surface of a known weight of the powder sample. Other ways of determining the surface area may be applied in the alternative.

In one embodiment, the anti-catabolic agent is a bisphosphonate that is pre-mixed with (and bound to) the calcium phosphate particles prior to mixing with the calcium sulphate powder. In a further embodiment, the calcium phosphate particles are crystalline hydroxyapatite particles. Alternatively, the anti-catabolic agent is added to and mixed with a pre-mixed calcium sulphate/calcium phosphate powder (a basis powder, e.g. a Cerament™ product).

In one other embodiment, bone active protein present in the powder is selected from the group comprising bone morphogenic proteins (BMPs), insulin-like growth factors (IGFs), transforming growth factor-βs (TGFβs), parathyroid hormone (PTH), strontium, sclerostine, cell factory derived proteins and ECM proteins. The bone active protein may be pre-mixed with the calcium sulphate hemihydrate powder, with the calcium phosphate powder or the basis powder.

The calcium sulphate powder, the calcium phosphate powder or the basis powder may also comprise one or more bioactive agents selected from antibiotics (including antifungal drugs), bone healing promotors, chemotherapeutics, cytostatics, vitamins, hormones, bone marrow aspirate, platelet rich plasma and demineralized bone. The at least one antibiotic agent may be selected from gentamicin, vancomycin, tobramycin, cefazolin, rifampicin, clindamycin and the antifungal drug is selected from the group comprising nystatin, griseofulvin, amphotericin B, ketoconazole and miconazole.

The calcium sulphate powder, the calcium phosphate powder or the basis powder may further comprising an X-ray contrast agent selected from water soluble non-ionic X-ray contrast agents and/or biodegradable X-ray contrast agents. The water soluble non-ionic X-ray contrast agent may be selected from iohexol, iodixanol, ioversol, iopamidol, iotrolane, metrizamid, iodecimol, ioglucol, ioglucamide, ioglunide, iogulamide, iomeprol, iopentol, iopromide, iosarcol, iosimide, iotusal, ioxilane, iofrotal, and iodecol.

Any of the additional bioactive agents / X-ray agents may be provided as powders or solutions and optionally added to any of the powders. Alternatively, the additional bioactive agents / X-ray agents may be encapsulated in synthetic polymeric microcapsules, bovine collagen particles, starch particles, dihydrate nidation particles, or the like before being mixed with any of the powders.

### Hardenable ceramic bone substitute paste

The present invention further concerns a hardenable ceramic bone substitute paste comprising a hardenable ceramic bone substitute powder as defined above and an aqueous liquid. The aqueous liquid may comprise any of the additives, including the bone active proteins and/or anti-catabolic agents discussed above. X-ray contrast agents and bioactive agents such as antibiotics are preferably dissolved in the aqueous liquid before mixing with the ceramic bone substitute powder. Alternatively, the additives, including the bone active proteins and/or anti-catabolic may be added to and mixed with the paste by delayed mixing as disclosed above. If one or more of the additives are interfering with the setting of the hardenable paste, such additives can advantageously be added to the paste by delayed mixing.

The liquid to dry powder ratio (L/P) in preparing the paste is in the range of 0.2 to 0.8 ml/g, such as 0.3 to 0.6 ml/g and preferably 0.4 to 0.5 ml/g.

In a preferred embodiment of the present invention, the hardenable ceramic bone substitute paste is prepared by mixed the powder(s), additives and liquid in a suitable bowl or in specially designed mixing devise (e.g. a Mixing and Injection Device (CERAMENT™ICMI) available from BONESUPPORT AB, Sweden or other mixing devices such as Optipac® from Biomet, US, used with or without vacuum) to be made ready for injection through a syringe (e.g. a specific injection device available from BONESUPPORT AB, Sweden). The additives may be part of the powder(s) or liquid or self-contained and added to together with the powder(s) and liquid. In a particular embodiment, one or more additives is/are added to the paste after the initial mixing of powder(s) and liquid in a "delayed mixing" process. It is important that addition and mixing of the additive(s) is/are performed before any setting reactions have started. Preferably, addition of additive(s) to the paste is performed within 2 to 4 minutes after initial mixing.

### Kit

The present invention also concerns kits for delivering all or some of the ingredients for use in the biphasic ceramic bone substitute according to the present invention. Such kits comprise:
i) a calcium sulphate hemihydrate powder;
ii) a calcium phosphate powder as defined in claim 3 or claim 4;
iii) a bone active protein as defined in any one of claims 5-6;
iv) an anti-catabolic agent which inhibits bone resorption as defined in claim 7; and optionally one or more of the following:
v) at least one further bioactive agent as defined above;
vi) a X-ray contrast agent as defined above;
vii) an accelerator for setting of the calcium sulphate, preferably calcium sulphate dihydrate or a salt such as NaCl;
viii) an accelerator for setting of the calcium phosphate, preferable particulate calcium phosphate particles and/or a phosphate salt such as disodium hydrogen phosphate (Na₂HPO₄);
ix) optionally an aqueous liquid.

The aqueous liquid may be distilled water, optionally comprising a salt and/or a buffer.

In one embodiment, the kit comprises a basis powder (x) comprising calcium sulphate hemihydrate powder (i) pre- mixed with the calcium phosphate powder (ii).

In another embodiment of the kit, the anti-catabolic agent (iv) is pre-mixed with at least a part of the calcium phosphate powder (ii), at least a part of the calcium sulphate hemihydrate powder (i), the basis powder (x), or the aqueous liquid (ix).

In yet another embodiment of the kit the bone active protein (iii) is pre-mixed with at least a part of the calcium sulphate hemihydrate powder (i), at least a part of the calcium phosphate powder (ii)), the basis powder (x), or the aqueous liquid ii).

In a further embodiment of the kit, the at least one further bioactive agent (v) as defined above is pre-mixed with the calcium phosphate powder (ii), the calcium sulphate hemihydrate powder (i), the basis powder (x), or the aqueous liquid (ix).

In yet a further embodiment of the kit the X-ray contrast agent (vi) as defined above is pre-mixed with the calcium phosphate powder (ii), the calcium sulphate hemihydrate powder (i), the basis powder (x), or the aqueous liquid (ix).

In another embodiment of the kit an accelerator for setting of the calcium sulphate (vii) as defined above is premixed with the calcium sulphate hemihydrate powder (i), the basis powder (x), or the aqueous liquid (ix).

In yet another embodiment of the kit an accelerator for setting of the calcium phosphate (viii) as defined above is pre-mixed with the calcium phosphate powder (ii), the calcium sulphate hemihydrate powder (i), the basis powder (x), or the aqueous liquid (ix).

Any of the additional bioactive agents / X-ray agents may be provided as such or in any of the powders or the liquid. Alternatively, the additional bioactive agents / X-ray agents may be encapsulated individually or in any suitable combination in water-soluble and/or biodegradable synthetic polymeric microcapsules, bovine collagen particles, starch particles and/or dihydrate nidation particles, or the like, and optionally mixed with any of the powders or the liquid.

According to the invention, the kit may further comprise mixing and injection devices, optionally including a syringe for injection. The kit may also comprise instructions for use.

In a further embodiment of the present invention, the kit further comprises a lining membrane for enclosing the synthetic grafts or closing the grafts to the outside, e.g. a biodegradable synthetic membrane or a collagen membrane as for example disclosed in WO2013185173. The synthetic graft may also be sealed with a protein in a solution that could be applied, i.a. as a spray, and thus support a surface healing. The covering protein may add additional benefits in preventing surface bacterial adherence and biofilm production.

In another aspect the present invention also concerns a method of treating patients with bone defects such as loss of bone due to, i.a. trauma, eradication of infection, resection of tumor lesions, delayed or nonunions and in primary or revision arthroplasties. In one embodiment, the method includes an insertion of one or more biphasic ceramic bone substitutes (grafts) according to the present invention into the bone lesion to be treated. In another embodiment, the method includes application of a paste of a hardenable biphasic ceramic bone substitute according to the present invention to the bone lesion to be treated. All bones in the animal or human body, including the spinal cord, bones of the hands, fingers, arms, feet, toes, lower or upper leg, knee, hip, ankle, elbow, wrist, shoulder, skull, jaw and teeth. The insertion of a biphasic ceramic bone substitute, for example in the form of a hardenable paste, may follow removal of bone, e.g. removal of broken bone, a bone tumor or infected bone tissue. In the case the substitute needs to be contained in the tissue around the graft or to prevent leakage to the surroundings or to cover an open wound, it may be beneficial or necessary to apply an artificial, e.g. polymeric, membrane. Such a membrane may be porous allowing body liquids and cells to flow to and from the porous graft and/or partially or fully sealed to the outside. After insertion of a biphasic ceramic bone substitute or the paste has hardened, the muscle tissue and skin may be repositioned or grafted over the artificial bone substitute.

### In vitro tests

### In-vitro immunogenicity

To see whether the Cerament™ products are immunogenic per se, a test involving RAW 264.7 macrophages, which are known to activate and secrete large amounts of cytokines when in contact with immunogenic materials, were selected. Ceramic discs prepared from Cerament™ products were seeded with murine macrophage cells RAW 264.7 and secretion of pro inflammatory cytokines like interleukin (IL)-1β, IL- 2, IL- 6 and tumor necrosis factor (TNF)-α was assessed over a period of 7- days using ELISA. The secretion of all cytokines (IL-1β, IL-2, IL-6 and TNF-α) is comparative with negative controls, and significantly lower than LPS (lipopolysaccharide) -treated positive controls. Application of Cerament™ in patients thus appears to give a very low if any immunological activity.

### In vitro osteoinductive effect

In some clinical cases extensive bone formation have been observed in the overlaying muscle covering surgically created bone defects treated with the hydroxyapatite/sulphate injectable mixture, CERAMENT™IBVF.

An in vitro model was designed to investigate the osteoinductive potential at the interface between muscle and bone substitute. Skeletal muscle cells were seeded on discs prepared from Cerament™BVF and from Cerament™G. Upon physiochemically characterizing Cerament™ using SEM, the porous structure was verified (Fig. 2A and B). Porous Cerament™ scaffold provides sufficient surface area for cellular attachment and also an efficient environment for exchange of nutrients, gases and other signaling molecules. Cells were uniformly distributed across the surface of the scaffold as observed from SEM and DAPI analysis (Fig. 2C-F). On both materials, skeletal muscle cellline C2CI2 differentiated into osteoblast like cells with expression of bone markers like runt-related transcription factor-2 (RUNX-2), collagen type 1 (COLI), osteocalcin (OCN), osteopontin (OPN) and bone sialoprotein (BSP). The cellular proliferation was similar on both the scaffolds with and without gentamycin indicating the addition of gentamycin to the scaffold in the model does not incur any negative effects on the cells (Fig. 3A). The scaffolds exhibited a gradual but suppressed proliferation of C2CI2 muscle myoblasts when compared with tissue culture plates. However, this difference in the proliferation behavior of cells on Cerament™ and 2D tissue culture plate might be due to differentiation of myoblast C2CI2 cells to osteoblast lineage. ALP is an important osteogenic marker and an 8-fold increase in the ALP activity (Fig. 3B) in the cells seeded on Cerament™ clearly demonstrated the osteoinductive behavior of the material irrespective of whether gentamycin was added or not. This was caused by the C2CI2 cells that differentiated to osteoblasts started going into maturation phase. It is known that ALP may acts as osteogenic cell formation predictor. RUNX2 is known to be an osteoblast specific transcription factor and a regulator of differentiation of osteoblast. The presence of other markers of mature osteoblast like COLI, OPN and OCN were detected by the 21^{st} day of cell seeding.

To mimic surgical conditions with leakage of extracellular matrix (ECM) proteins and growth factors from artificial grafts, bone cells ROS17/2.8 were cultured in a bioreactor and the secreted growth factors and ECM proteins were harvested. Harvested cell culture produced bone active proteins were measured using ELISA and bone morphogenic protein-2 (BMP-2, 8.4 ± 0.8 ng/mg) and BMP-7 (50.6 ± 2.2 ng/mg) were found. In vitro, the harvested bone active proteins induced differentiation of skeletal muscle cells L6 towards an osteogenic lineage, which stained positive for bone markers.

Based on the above results, it was found that bone formation can be synergistically enhanced by release of growth factors and/or ECM proteins capable of inducing osteoblast differentiation from and present in biphasic ceramic bone substitute.

### In-vitro BMP-2 release

A Cerament™BVF-rhBMP-2 paste was prepared by mixing, transferred to a syringe and solid discs were prepared in a mold. Each disc containing 2µg rhBMP-2 was immersed in 1 mL saline and placed in an incubator at 37 °C. At different time point over a period of 7-days, 50 µl of saline from the supernatant was collected and analysed and the protein concentration calculated. A constant release of BMP-2 from Cerament™BVF was observed over a period of 7-days with nearly 90% of rhBMP-2 released after 7-days.

### In-vitro ZA release

Release of bisphosphonate (zoledronic acid (ZA) was used as an example) from a biphasic ceramic bone substitute was investigated in Cerament™ with and without gentamicin. Cerament™BVF-ZA paste and Cerament™G-ZA paste were prepared by mixing each of the Cerament™ powders with ZA and a liquid. The discs were produced by transferred the pastes to a mold using a syringe and the solid discs were left to set. Saline was added to the discs and they were incubated at physiological conditions. At different time point over a period of 7-days, a sample of the medium was collected and analysed. To assess the release of ZA from each Cerament™+ZA disc at different time points, the collected supernatants were added to A549 cells and cell viability was calculated after an incubation of 48 h using MTT assay. The concentration of ZA was calculated from a standard curve.

After a period of 7-days, the amount of ZA released from solid Cerament™ discs was nearly 10% of the total ZA loaded. No difference in ZA-release was seen between Cerament™ with and without gentamicin. The cytotoxic effect of ZA released from Cerament™BVF and Cerament™G discs on A549 cells indicated a decrease in cell viability at increasing time points.

### In vivo testing (ectopic (muscle) bone model)

Discs were produced from Cerament™ products mixed with recombinant human (rh) BMP-2 alone or with rhBMP-2 together with ZA and implanted in 7 week old rats. In a modified ectopic bone model, the implants were inserted in the abdominal muscle by performing a single blunt dissection of the abdominal muscle The modified ectopic bone model is unique in using the unstressed abdominal muscle, which results in an increased resorption of bone cells by osteoclasts compared to an earlier study, where the grafts are placed next to an existing bone in the hip joint on the dorsal side, and thus more likely is influenced by local release and stimulation from the underlying bone which will affect the level bone being built and the tested anti-catabolic agents such as bisphosphonates as well as the growth hormones (WO 2012/094708). In one group the test animals received two discs of only Cerament™BVF in the left side of the abdominal midline per animal while the right side of the midline was used to implant two discs of Cerament™BVF + rhBMP-2 per animal. In another group, the animals received two discs of only Cerament™BVF and two discs of Cerament™BVF + rhBMP-2+ ZA in a similar manner. The scaffolds emerging over time from the discs were left in the animals for 4 weeks. Analysis for bone formation was done using X-ray followed by three-dimensional analysis of mineralized tissue volume using micro computed tomography (micro-CT) and electron microscopy. The type of cells within the scaffold was analyzed using histology (Hematoxylin and eosin (H&E)).

Examination of the animals sacrificed after 4 weeks showed that the scaffolds from Cerament™BVF discs loaded with rhBMP-2 and ZA are denser than the scaffolds from Cerament™BVF discs loaded with rhBMP-2 only and scaffolds from Cerament™BVF discs. Micro-CT results show that the mineralized tissue volume was significantly higher in the Cerament™BVF disc group loaded with a combination of rhBMP-2 and ZA than in the group loaded with rhBMP-2 and the group with Cerament™BVF discs. The group loaded with a combination of rhBMP-2 and ZA had significantly higher mineral volume than the Cerament™BVF + rhBMP-2 group. Histologically, the samples that were loaded with rhBMP-2+ ZA had developed a cortical shell around the scaffold with islands of trabecular bone already visible within the scaffold, while the Cerament™BVF + rhBMP-2 group showed signs of osteoclastic resorption with visible fatty marrow. This is clearly visualized by the electronmicroscopy.

### EXAMPLES

The content of Cerament™ compositions used in the examples is given in Table 1.

In all of the examples "saline" means a NaCl solution containing 9 mg NaCl/mL water unless stated otherwise.

### Example 1

### In-vitro immunogenicity

Cerament™BVF paste was prepared according to the manufacturer's instruction and used to prepare discs (diameter: 8 mm; height: 2 mm) which sat before 20 minutes. The discs were seeded with a total of 1x10⁵ murine macrophage cells RAW 264.7 and secretion of pro inflammatory cytokines like interleukin (IL)-1β, IL- 2, IL-6 and tumor necrosis factor (TNF)-α was assessed over a period of 7- days using ELISA. As positive control, RAW 264.7 cells were treated with immunogenic lipopolysaccharide (LPS).

The secretion of all cytokines (IL-1β, IL-2, IL-6 and TNF-α) was comparable with the negative control (2D-TCP) and significantly lower than the LPS treated positive control (2D-TCP + LPS) with p-values < 0.0001 in all cases (Figure 1A-D).

### Example 2

### In vitro osteoinduction

### Material preparations for the in vitro experiments

Two types of bone substitute products, CERAMENT™IBVF and CERAMENT™IG, were mixed as per supplier's guidelines (Bone Support AB, Lund, Sweden) to form a homogenous paste. The paste was poured in a disc shape mold with 8 mm diameter and 2 mm height and allowed to set for 30 min.

3-(4,5- dimethylthiazol-2-yl)-2,5-diphenyltetrazolium bromide (MTT), Sigmafast pNPP, Dulbecco's Modified Eagle's Medium- High glucose (DMEM-HG), Fetal bovine serum (FBS), antibiotic cocktail, Trizol reagent and primers for real time polymerase chain reaction (RT-PCR) was purchased from Sigma Aldrich, MA, USA. Mouse COLI, OCN, RUNX-2, OPN were purchased from Santa Cruz Biotechnology, Inc., CA, USA and Sigma Chemical company, MA, USA. Rat COLI, OCN, OPN and bone sialoprotein (BSP) antibodies, DRAQ5, alexa flour 488 (AF-488) were procured from Abcam, Cambridge, U.K. RT-PCR reagents were purchased from Thermo scientific, USA. Rat BMP-2 and BMP-7 ELISA kits were purchased from Abnova Inc., Taiwan and Qayee Bio, China respectively. All other reagents were of high purity purchased from recognized suppliers.

### Cell culture

Mouse myoblast C2CI2 cells were cultured in the Dulbecco's Modified Eagle's Medium (DMEM) supplemented with 10% (v/v) fetal bovine serum (FBS) and antibiotics. Cells were kept in an incubator having 95% air and 5% C02. For the proliferation and functionality experiments, 1 x 10⁵ cells were seeded onto the Cerament™ discs while for immunofluorescence staining and reverse transcription polymerase chain reaction (RT-PCR), 1 x 10⁶ cells were seeded onto the Cerament™ discs. Rat skeletal muscle myoblast cellline L6 was cultured in DMEM with high glucose with 10% (v/v) FBS and 1% (v/v) antibiotic cocktail consisting of penicillin-streptomycin. Cells were passaged at 80% confluence and were used at 2^{nd} passage after revival. Cell viability before experiments was evaluated using the trypan blue exclusion method.

In order to mimic in vivo conditions that lead to bone formation in the muscle tissue, osteoblast cell factory derived proteins were harvested from an expanded cell culture of ROS 17/2.8 osteoblastic cells. Cells were allowed to proliferate in culture flasks supplemented with complete medium and 5% (v/v) serum for a period of 3 days. The secreted bone active proteins in the medium were collected while the cells were passaged again to repeat the procedure.

In order to ensure transdifferentiation of muscle cells into osteoblast like cells, the rat muscle cell line L6 was used. The cells were allowed to grow to 80% confluence after which they were either supplied with low serum (5% v/v) complete medium or a mixture of complete medium (low serum) and harvested osteoblast cell factory medium in an equal ratio by volume. The cells were allowed to grow for a period of 10 or 12 days and were analyzed using different techniques to confirm a shift in their phenotype.

### Statistical analysis

Data from the MTT and ALP assay were analyzed using unpaired t-test. p < 0.05 was considered to be significant. Data from MTT assay and myotube numbers for cell factory experiments were analyzed using non-parametric, multiple t-test and p < 0.05 was considered statistically significant. Data is represented in triplicates with mean and standard deviation.

### Microscopic analysis

Surface morphology of the materials and adherence of the C2C12 cells on the surface of Cerament™ discs were analyzed using scanning electron microscopy. Materials were dehydrated by gradient ethanol treatment. Further, samples were vacuum dried overnight. For analyzing the cell adherence on the Cerament™ surface, cells were seeded on both the materials i.e., with gentamicin and without gentamicin. The cells were allowed to grow for three days. Thereafter, glutaraldehyde (2.5 %) was used to fix all the cells on the surface. Steps following fixation were the same as were used for sample preparation for surface morphology analysis. Furthermore, attachment of cells on the Cerament™ discs were analysed using 4', 6-diamidino-2-phenylindole (DAPI) staining.

The surface morphology of both Cerament™ discs with and without gentamycin showed porous structure with size of the pores at the material surface in the range of 1-10 µm (Figure 2A and B). Cells were present on the surface of the Cerament™ discs after 3 days of seeding (Figure 2C and D). The cells were attached and homogenously distributed on both discs, with and without gentamicin (Figure 2E and F). This was further confirmed by staining of the cells with DAPI that revealed similar type of distribution pattern where cells seeded were adhered and evenly distributed on the surface of material.

### Cell proliferation assay

Cell proliferation on both the materials was evaluated using MTT assay at regular time intervals. Briefly, the DMEM media in the wells was removed, and cell seeded inorganic discs were washed using phosphate buffer saline (PBS). Thereafter, DMEM media, without FBS, containing MTT (0.5 mg/ml) was added in the wells and incubation of 5 h was done. Further, this solution was removed and dimethyl sulfoxide (DMSO) was added. The samples were incubated for 20 min at 37 °C. The colored solution formed was collected and absorbance was measured spectrophotometrically at 570 nm. Cell proliferation analysis in the cell factory experiments using L6 cells was done in a similar manner and a cell density of 5 x 10⁴ cells/well was used. The proliferation of myotubes was analyzed by microscopy and multinucleated and elongated cells were considered to be myotubes.

Similar results were observed in both Cerament™ materials with or without gentamicin (Figure 3A). After the initial increase in the cell population until the 7^{th} day, the cell proliferation was suppressed and a decrease in cell population was observed at the later time points. On the other hand, in case of two-dimensional polystyrene tissue culture plates, taken as a control, increase in cell proliferation was observed till the 14^{th} day, thereafter proliferation starts declining. Cells seeded on both the materials showed similar profile of cell proliferation. No statistically significant difference in cell proliferation was reported (p>0.05). However, better cell proliferation was reported in the polystyrene tissue culture plate compared to the Cerament™ discs.

### Alkaline phosphatase assay

Sigma fast para-Nitrophenylphosphate (pNPP) tablets were used to prepare pNPP substrate solution, using protocol provided by the manufacturer. The media was removed from the wells and samples were washed using PBS buffer. The samples were then incubated with para-nitrophenylphosphate (pNPP) substrate solution for 2 h in the C0₂ incubator at 37 °C and absorbance was measured at 405 nm.

The material with and without gentamycin showed increase in ALP amounts by the 14^{th} day of cell seeding (Figure 3B). Thereafter, values of ALP start decreasing with time. Within a time period of 14 days, cells seeded on both the Cerament™ materials showed an eight-fold increase in ALP level when compared to polystyrene controls (p<0.05). There was no statistically significant difference in the level of ALP shown by both the materials (p>0.05). On the other hand, the level of ALP by the cells seeded on the polystyrene plate was less as compared to ALP level of cells seeded on Cerament™ materials.

### Immunofluorescence staining for osteogenic markers

The differentiation potential of the materials were observed using immunofluorescence staining. The cells were stained to detect the presence of different markers like runx2, osteopontin, osteocalcin and collagen type I (COLI) over the period of 21 days.

Immunofluorescence staining showed the presence of Runx2 by the 7^{th} day of cell seeding on the Cerament™ disc (Figure 4A-C). The presence of other markers of mature osteoblasts like COLI (Figure 4D-F), OCN (Figure 4G-I) and OPN (Figure 4J-L) were detected by the 21^{st} day of cell seeding.

To confirm the transdifferentiation of L6 muscle cells into osteoblast like cells, cells in both groups were immunostained for various osteoblastic markers like collagen type I (COLI), osteocalcin (OCN), osteopontin (OPN) and bone sialoprotein (BSP). Cells were allowed to grow in culture flasks for a period of 10 days in complete medium with osteoblast harvested bone active proteins or low serum. The cells were trypsinized and seeded on 4-well chamber slides and allowed to proliferate with same medium further for 48 h. At the day of staining, cells were fixed using 4% formaldehyde for 10 min followed by membrane permeabilization using 0.1% (v/v) triton X-100 for 5 minutes. Later cells were blocked using 5% goat serum for 1 h and incubated with respective primary antibodies for 2 h at room temperature. Slides were washed with PBST five times followed by incubation in secondary antibody (AF-488 labeled) for 1 h. The slides were counterstained using DRAQ5 for 5 min and washed twice for 5 min each. The slides were eventually cleared, mounted and allowed to dry overnight before analysis. The cells were analyzed on Zeiss confocal microscope at different magnifications.

Figure 6 shows the immuno positive and transdifferentiated muscle cells transformed into osteoblast like cells. The cells in the treated groups expressed osteogenic proteins like COLI, OCN, OPN and BSP at day 12 (Figure 6A-D). The cells in the control group fused together to form myotubes and did not express osteogenic proteins.

### RNA extraction and RT- PCR

The discs of Cerament™ with and without gentamicin were seeded with C2C 12 cells at a concentration of 1x10⁶ cells/disc. The RNA was extracted using Trizol reagent, after in vitro culturing of cell seeded discs for the time period of 7 and 21 days. Cell seeded discs were transferred from multiwell plate to microtubes after adding 1 ml of Trizol reagent. Thereafter, RNA was isolated by following the protocol supplied by the manufacturer. Complimentary DNA (cDNA) was synthesized by incubating isolated RNA (20 µl) with 1 µl of oligodT at 75 °C for 5 min followed by incubation on ice for 5 min. To this, cDNA mix having 4 µl of buff RT, 1 µl of RTase, 0.5 µl of RI (RNase inhibitor) and 2 µl of dNTP mix was added. RT-PCR was conducted to evaluate the expression of various genes of the osteogenic lineage such as RUNX2, COLI, BSP and OCN. The primer sequences of the genes are obtained from previous work and listed in Table 2. As an endogenous control, expression of Glyceraldehyde 3-phosphate dehydrogenase (GAPDH) was determined. Consecutive thermal cycle was used for DNA amplification. Products of RT-PCR were resolved on a 2.0% agarose gel stained using ethidium bromide

**Table 2.**

| | Primer | Sequences |
|---|---|---|
| 1. | RUNX2 | F:TTTAGGGCGCATTCCTCATC |
| | | R:TGTCCTTGTGGATTAAAAGGACTTG |
| 2. | BSP | F:CACCCCAAGCACAGACTTTT |
| | | R: GTTCCTTCTGCACCTGCTTC |
| 3. | COLI | F:GAGGCATAAAGGGTCATCGTGG |
| | | R:CATTAGGCGCAGGAAGGTCAG |
| 4. | OCN | F:GAACAGACTCCGGCGCTA |
| | | R:AGGGAGGATCAAGTCCCG |
| 5. | GAPDH | F:TCCACTCACGGCAAATTCAACG |
| | | R:TAGACTCCACGACATACTCAGC |

Results showed presence of RUNX2 by the 7^{th} day (Figure 5M) and presence of other osteoblastic marker such as COLI, BSP and OCN by the 21^{st} day of cell seeding (Figure 5N).

### Morphological changes using light microscopy and Hematoxylin and Eosin staining

The transdifferentiation of muscle cells with the addition of osteoblast harvested bone active proteins was analyzed over a period of several days. Morphological analysis was performed using both light microscopy and H&E staining. Culture flasks were directly monitored using a light microscope at different magnifications. In order to perform H&E staining, cells were grown in 4-well chamber slides and were fixed with 4% (w/v) formaldehyde for 10 min. Cells were hydrated with reducing ethanol gradient and stained with Hematoxylin for 5 min. Excessive stain was washed using running water followed by counterstaining with Eosin for 2 min. The slides were cleared in xylene for 5 min, mounted and dried overnight before imaging.

A time course morphological differences were observed in cells treated with bone active proteins and the control groups. The cells in the control groups can be seen as elongated from as early as day 1 until the end of the experiment (Figure 6E and F) when compared with cells in the treated groups (not shown). Hematoxylin and eosin staining clearly depicts the structural differences between the cells with and without treatment of growth factors (Figure 6F and H). In case of controls, muscle cells differentiate into fused myotubes possessing a number of nuclei while the cells in the group treated with growth factors remain uninucleated throughout the experiment (Fig 6F and H). Moreover the size of the cells is much smaller and possesses osteoblast like morphology in case of treated cells.

### Cell viability and myotube number

No significant difference in proliferation profile of cells was observed (Figure 6I). On the contrary, cells in the control groups fused to form myotubes with multiple nuclei. The number of myotubes that could be observed over a period of 7 days was also analyzed. The number of myotubes in the control group kept increasing over time (p< 0.05), however in the treated group very few myotubes were observed on day 1 and the number reached zero after day 3 indicating complete suppression of myotube formation (Figure 6J).

### Osteoblast cell factory composition

With an attempt to detect various pro-osteoblastic proteins in the ROS 17/2.8 cell factory the harvested cell factory proteins were dialyzed against ultrapure water for a period of 48 hr. using a 8 kDa dialysis membrane. After dialyzing, the proteins were concentrated using freeze-drying for a period of 48 hr. The dried protein fraction so obtained was later analyzed using ELISA for the detection and measurement of two important bone active molecules BMP-2 and BMP-7.

The presence of the two most common osteoinductive proteins, BMP-2 and BMP-7 responsible for osteogenic differentiation of various mesenchymal cells into osteoblastic lineages were confirmed. The detection of osteogenic proteins was performed using ELISA and the respective concentrations of BMP-2 and BMP-7 in the cell factory were 8.4 ± 0.8 ng/mg and 50.6 ± 2.2 ng/mg of the harvested protein fraction.

### Example 3

### In-vitro BMP-2 release

1 g of Cerament™BVF was mixed with 0.406 ml CERAMENT™IC-TRU. The Cerament™BVF paste was mixed rigorously for 30 seconds followed by waiting for 30 seconds and this was continued until 2.5 minutes. A stock solution of rhBMP-2 (Medtronic) containing 40 µg rhBMP-2 was prepared by dissolving it in 40 µL saline (9 mg NaCl/mL). At 2.5 minutes 24 µl of this rhBMP/saline stock solution was rigorously mixed into the pre-mixed Cerament™BVF paste. After complete mixing of the rhBMP-2 solution into the Cerament™BVF paste, the rhBMP/Cerament™BVF paste was transferred to a syringe and 12 discs were made (diameter: 5±0.1 mm; height: 1.5±0.05 mm). All discs were set before 20 min. The weight of the discs was 46±3.2 mg and each disc contained 2µg rhBMP-2.

Each disc was immersed in 1 mL saline and placed in an incubator at 37 °C. At each time point (Day 1, 3, 5 and 7) 50 µl of the supernatant was collected for analysis and 50 µl of fresh saline was added. The protein concentration was calculated using ELISA over a period of 7-days. Figure 7 shows that a constant release of BMP-2 from solid Cerament™BVF was observed over a period of 7-days with nearly 90% of rhBMP-2 released after 7-days.

### Example 4

### In-vitro ZA release

For the in-vitro zoledronic acid (ZA) release test a total of 12 discs were prepared; 6 discs were prepared from Cerament™BVF powder and 6 discs were prepared from Cerament™G powder:
500 mg of Cerament™BVF was mixed with 148.64 µl CERAMENT™IC-TRU. The sample was mixed rigorously for 30 seconds followed by waiting for 30 seconds and this was continued until 2.5 minutes. At 2.5 minutes 67.5 µl zoledronic acid solution (54 µg ZA, Zometa (4mg/5ml), Novartis) was added to the Cerament™BVF paste. The Cerament™BVF + ZA paste was mixed for 30 more seconds and 6 discs were prepared (diameter: 5±0.1 mm; height: 1.5±0.05mm; 9 µg ZA). All discs were set before 20 min.

500 mg of Cerament™G powder was mixed with 148.64 µl saline containing 6.6 mg gentamicin. The sample was mixed rigorously for 30 seconds followed by waiting for 30 seconds and this was continued until 3.5 minutes. At 3.5 minutes 67.5 µl zoledronic acid solutions (54 µg ZA, Zometa, Novartis) was added to the Cerament™G paste. The Cerament™G + ZA paste was mixed for 30 more seconds and 6 discs were prepared (diameter: 5±0.1 mm; height: 1.5±0.05mm; 9 µg ZA). All discs were set before 20 min.

Saline was added to the discs and they were incubated at physiological conditions. At each time point, a sample of the medium was collected and stored for further analysis. To assess the release of ZA from each Cerament™BVF/G + ZA disc at different time points, the collected supernatants were added to A549 cells and cell viability was calculated after an incubation of 48 hours using MTT assay. The concentration of ZA was then calculated from the obtained standard curve.

In-vitro statistical analysis was performed using multiple t-test (Prism 6) with data represented in triplicates with mean and standard deviation.

After a period of 7-days, the amount of ZA released from the Cerament™BVF and Cerament™G discs was nearly 10% of the total ZA loaded (Figure 8; BVF and G groups taken together, as no difference was seen between the Cerament™BVF and Cerament™G discs). The cytotoxic effect of ZA released from Cerament™ discs on A549 cells indicates a decrease in cell viability at increasing time points (Figure 9; BVF and G groups taken together, as no difference was seen between the Cerament™BVF and Cerament™G discs).

### Example 5

### In-vivo muscle pouch model

The study was approved by the local authority for use of laboratory animals (permit M 124-14). Discs of Cerament™BVF, Cerament™BVF + rhBMP-2 and Cerament™BVF + rhBMP-2 and ZA were produced as follows:

### The Cerament™BVF discs:

1 g of Cerament™BVF was mixed with 0.43 mL of a iohexol-solution comprising 162 µl saline and 268 µl CERAMENT™IC-TRU and rigorously mixed for 30 seconds followed by waiting for 30 seconds and this was repeated until 2.5 min. The total liquid used was 430 µl for 1 g Cerament™ powder, which gives 480 µl paste with a liquid/powder ratio of 0.43 ml/g. The paste was used to prepare 12 cylindrically discs (diameter: 5 mm; height: 2 mm; weight: 47.6± 3 mg) in a sterile mold (40 µl paste/cylinder). Each disc which contained 83.33 mg Cerament™BVF, 22.33 µl CERAMENT™IC-TRU and 13.5 µl saline and sat before 20 minutes.

### The Cerament™BVF + BMP discs:

In the "Cerament™BVF +BMP" group a stock solution of BMP was initially prepared by dissolving 120 µg of rhBMP-2 (Medtronic) in 162 µl of saline. 1 g of the Cerament™BVF was mixed with 268 µl CERAMENT™IC-TRU and rigorously mixed for 30 seconds followed by waiting for 30 seconds and repeated mixing and pausing until 2.5 minutes to obtain a paste. At 2.5 minutes, the 162 µl BMP/saline solution (containing 120 µg rhBMP-2) was added to the paste and rigorously mixed for another 30 seconds. The total liquid used was 430 µl for 1 g Cerament™BVF powder, which gives a liquid/powder ratio of 0.43 ml/g. A final volume of 480 µl paste was obtained containing 120 µg rhBMP-2 and used to prepare 12 discs of the same size as above, each with a volume of 40 µl BMP/Cerament™BVF paste. Each disc contained 83.33 mg Cerament™BVF, 22.33 µl CERAMENT™IC-TRU, 13.5 µl saline and 10 µg rhBMP-2. The discs sat before 20 minutes.

### The Cerament™BVF + BMP+ ZA discs:

A solution of rhBMP-2 was prepared by dissolving 120 µg rhBMP-2 (Medtronic) in 12 µl of saline. 150 µl of a ZA-solution (120 µg ZA, Novartis) was added and mixed with the rhBMP-2 solution. A total volume of 162 µl of ZA (120 µg) and rhBMP-2 (120 µg) in saline was achieved. 1 g of Cerament™BVF was mixed with 268 µl CERAMENT™IC-TRU and the paste was rigorously mixed for 30 seconds followed by waiting for 30 seconds and mixing and pausing were repeated until 2.5 minutes to prepare a paste. At 2.5 minutes the 162 µl ZA+rhBMP-2 solution was added to the paste and mixed for 30 seconds more to homogenize the contents. A final volume of 480 µl was obtained and used to produce 12 discs as described above, each with a volume of 40 µl ZA/BMP/Cerament™ paste. Each disc contained 83.33 mg Cerament™BVF, 22.33 µl CERAMENT™IC-TRU, 13.5 µl saline, 10 µg rhBMP-2 and 10 µg ZA. The discs sat before 20 minutes.

Discs comprising Cerament™BVF, Cerament™BVF + rh-BMP-2 and Cerament™BVF + rh-BMP-2 + ZA prepared as described above were implanted in 7 week old Sprague Dawley rats. The implants were inserted in the abdominal muscle by performing a single blunt dissection of the abdominal muscle. In one group, five animals received two discs containing only Cerament™BVF in the left side of the abdominal midline per animal while the right side of the midline was used to implant two discs containing Cerament™BVF + BMP-2 per animal. In a second group, 5 animals received two discs containing Cerament™BVF and two discs containing Cerament™BVF + BMP-2+ ZA in a similar manner. The scaffolds emerging over time from the discs were left in the animals for 4 weeks. Analysis for bone formation was done using X-ray followed by three-dimensional analysis of mineralized tissue volume using micro computed tomography (micro-CT). The type of cells within the scaffold was analyzed using histology (H&E).

In-vivo statistical analysis was performed using student t-test with n=5 (mean and SD). P- value < 0.05 was considered to be significant.

As seen in Figure 10, radiographic examination of the animals after 4 weeks showed that the scaffolds from Cerament™BVF discs loaded with rhBMP-2 and ZA are denser than the scaffolds from Cerament™BVF discs loaded with rhBMP-2 only. Scaffolds from Cerament™BVF discs loaded with rhBMP-2 are denser than scaffolds from Cerament™BVF discs. Micro-CT results show that the mineralized tissue volume was significantly higher in the Cerament™BVF disc group loaded with a combination of rhBMP-2 and ZA and in the group loaded with rhBMP-2 when compared to the group of only Cerament™BVF discs (p< 0.01) (Figures 11-13). The group loaded with a combination of rhBMP-2 and ZA had significantly higher mineral volume than the Cerament™BVF + BMP-2 group (p<0.01). Histologically, the samples that were loaded with rhBMP-2+ ZA had developed a cortical shell around the scaffold with islands of bridging trabecular bone already visible within the scaffold, while the Cerament™BVF+ BMP-2 group showed signs of osteoclastic resorption with visible fatty marrow (Figure 11).

## Claims

1. A biphasic ceramic bone substitute comprising:
a. a calcium sulphate phase;
b. a calcium phosphate phase;
c. at least one bone active protein, and
d. at least one anti-catabolic agent which inhibits bone resorption selected from bisphosphonic acids and bisphosphonates,
wherein the at least one bone active protein is comprised in the calcium sulphate phase and the at least one anti-catabolic agent is comprised in the calcium phosphate phase.

2. A biphasic ceramic bone substitute according to claim 1, wherein the calcium sulphate is calcium sulphate dihydrate.

3. A biphasic ceramic bone substitute according to claim 1 or claim 2, wherein the calcium phosphate is selected from the group consisting of α-tricalcium phosphate, hydroxyapatite, tetracalcium phosphate and β-tricalcium phosphate.

4. A biphasic ceramic bone substitute according to claim 3, wherein the calcium phosphate phase is composed of hydroxyapatite, preferably crystalline hydroxyapatite particles.

5. A biphasic ceramic bone substitute according to any one of claims 1-4, wherein the bone active protein is selected from the group comprising bone morphogenic proteins (BMPs), insulin-like growth factors (IGFs), transforming growth factor-βs (TGFβs), parathyroid hormone (PTH), sclerostin, cell factory derived bone active proteins and extracellular matrix (ECM) proteins.

6. A biphasic ceramic bone substitute according to claim 5, wherein the bone active protein is a bone morphogenic protein (BMP), such as BMP-2, preferably rhBMP-2, and/or BMP-7, preferably rhBMP-7.

7. A biphasic ceramic bone substitute according to any one of claims 1-6, wherein the anti-catabolic agent is a bisphosphonate selected from the group comprising etidronate, clodronate and tiludronate, or the group comprising pamidronate, neridronate, olpadronate, alendronate, ibandronate, risedronate and zoledronate.

8. A biphasic ceramic bone substitute according to any one of claims 1-7 comprising at least one further bioactive agent selected from antibiotics, antifungal drugs, bone healing promotors, chemotherapeutics, cytostatics, vitamins, hormones, bone marrow aspirate, platelet rich plasma and demineralized bone.

9. A biphasic ceramic bone substitute according to claim 8 comprising at least one antibiotic selected from gentamicin, vancomycin, tobramycin, cefazolin, rifampicin, clindamycin and the antifungal drug is selected from the group comprising nystatin, griseofulvin, amphotericin B, ketoconazole and miconazole.

10. A biphasic ceramic bone substitute according to any one of claims 1-9 further comprising an X-ray contrast agent selected from water soluble non-ionic X-ray contrast agents and/or biodegradable X-ray contrast agents.

11. A biphasic ceramic bone substitute according to claim 10, wherein the water soluble non-ionic X-ray contrast agent is selected from iohexol, iodixanol, ioversol, iopamidol, iotrolane, metrizamid, iodecimol, ioglucol, ioglucamide, ioglunide, iogulamide, iomeprol, iopentol, iopromide, iosarcol, iosimide, iotusal, ioxilane, iofrotal, and iodecol.

12. A biphasic ceramic bone substitute according to any one of claims 1-11, wherein the calcium sulphate to calcium phosphate ratio (w/w) is from 5:95 to 95:5, from 10:90 to 90:10, from 20:80 to 80:20, from 30:70 to 70:30, or from 40:60 to 60:40.

13. A biphasic hardenable ceramic bone substitute paste for producing a biphasic ceramic bone substitute according to any one of claims 1-12, comprising:
a. a calcium sulphate hemihydrate powder;
b. a calcium phosphate powder, where the calcium phosphate is selected from α-tricalcium phosphate, hydroxyapatite, tetracalcium phosphate and β-tricalcium phosphate.;
c. a bone active protein;
d. an anti-catabolic agent which inhibits bone resorption selected from bisphosphonic acids and bisphosphonates;
e. optionally an accelerator for setting of calcium sulphate in an aqueous solution, preferably selected from calcium sulphate dihydrate and an inorganic salt, such as NaCl;
f. optionally an accelerator for setting of calcium phosphate in an aqueous solution, preferably particulate calcium phosphate and/or a phosphate salt, such as Na₂HPO₄; and
g. an aqueous liquid.

14. A biphasic hardenable ceramic bone substitute paste according to claim 13, wherein the anti-catabolic agent is pre-mixed with and bound to the calcium phosphate particles in the powder.

15. A biphasic hardenable ceramic bone substitute paste according to claim 13 or claim 14, wherein the calcium phosphate particles are crystalline hydroxyapatite particles.

16. A biphasic hardenable ceramic bone substitute paste according to any one of claims 13-15, further comprising a bioactive agent selected from antibiotics, antifungal drugs, bone healing promoters, chemotherapeutics, cytostatics, vitamins, hormones, bone marrow aspirate, platelet rich plasma and demineralized bone.

17. A biphasic hardenable ceramic bone substitute paste according to any one of claims 13-16 further comprising an X-ray contrast agent selected from water soluble non-ionic X-ray contrast agents and/or biodegradable X-ray contrast agents.

18. A biphasic hardenable ceramic bone substitute paste according to any one of claims 13-17, wherein the bone active protein and/or the anti-catabolic agent and/or the further bioactive agent and/or the X-ray contrast agent is/are mixed with the aqueous liquid before being mixed with the powder(s).

19. A biphasic hardenable ceramic bone substitute paste according to any one of claims 13-18, wherein the paste is injectable.

20. A biphasic hardenable ceramic bone substitute paste according to any one of claims 13-19, wherein the calcium sulphate to calcium phosphate ratio (w/w) is from 5:95 to 95:5, from 10:90 to 90:10, from 20:80 to 80:20, from 30:70 to 70:30, or from 40:60 to 60:40; and the liquid to dry powder ratio is from 0.2 to 0.8, preferably from 0.3 to 0.6.

21. A biphasic hardenable ceramic bone substitute paste according to any one of claims 13-20 for use in the treatment of a disorder of supportive tissue in a human or non-human subject involving an initial stimulation of bone cell activation and growth and a suppression of premature resorption of the newly formed bone.

22. A kit for producing a biphasic hardenable ceramic bone substitute paste according to any one of claims 13-20, or a biphasic ceramic bone substitute according to any one of claims 1-12, comprising the following components:
i) a calcium sulphate hemihydrate powder;
ii) a powder of a calcium phosphate as defined in claim 3 or claim 4;
iii) a bone active protein as defined in claim 5 or claim 6;
iv) an anti-catabolic agent as defined in claim 7;
v) optionally at least one further bioactive agent as defined in claim 8 or claim 9;
vi) optionally an X-ray contrast agent as defined in claim 10 or claim 11;
vii) optionally an accelerator for setting of the calcium sulphate, preferably calcium sulphate dihydrate or an inorganic salt, such as NaCl;
viii) optionally an accelerator for setting of the calcium phosphate, preferable particulate calcium phosphate and/or a calcium phosphate salt, such as Na₂HPO₄; and
ix) optionally an aqueous liquid, such as water.

23. A kit according to claim 22, further comprising:
a. a mixing and/or injection device(s), and/or
b. instructions for use.

24. A kit according to claim 22 or claim 23, further comprising a biodegradable synthetic membrane or a collagen membrane.

25. A biphasic ceramic bone substitute according to any one of claims 1-12, a biphasic ceramic bone substitute paste according to any one of claims 13-21 or a kit according to any one of claims 22-24, wherein one or more of the additive is/are provided as encapsulated individually or in any combination(s) in water-soluble and/or biodegradable synthetic polymeric microcapsules, bovine collagen particles, starch particles, dihydrate nidation particles.

26. A biphasic ceramic bone substitute according to any one of claims 1-12 and 25, or a biphasic ceramic bone substitute paste according to any one of claims 13-21 for use in the treatment of a patient with a bone defect, such as loss of bone due to, i.a. trauma, eradication of infection, resection of tumor lesions, delayed or nonunions and in primary or revision arthroplasties said treatment comprising inserting one or more biphasic ceramic bone substitute(s) (grafts) or said biphasic hardenable biphasic ceramic bone substitute paste at the place of removed bone.

## Patentansprüche

1. Biphasischer keramischer Knochenersatz, umfassend:
a. eine Calciumsulfatphase;
b. eine Calciumphosphatphase;
c. mindestens ein knochenaktives Protein und
d. mindestens ein antikatabolisches Mittel, das die Knochenresorption hemmt, ausgewählt aus Bisphosphonsäuren und Bisphosphonaten,
wobei das mindestens eine knochenaktive Protein in der Calciumsulfatphase enthalten ist und das mindestens eine antikatabolische Mittel in der Calciumphosphatphase enthalten ist.

2. Biphasischer keramischer Knochenersatz nach Anspruch 1, wobei das Calciumsulfat Calciumsulfatdihydrat ist.

3. Biphasischer keramischer Knochenersatz nach Anspruch 1 oder Anspruch 2, wobei das Calciumphosphat ausgewählt ist aus der Gruppe, bestehend aus α-Tricalciumphosphat, Hydroxyapatit, Tetracalciumphosphat und β-Tricalciumphosphat.

4. Biphasischer keramischer Knochenersatz nach Anspruch 3, wobei die Calciumphosphatphase aus Hydroxyapatit, vorzugsweise kristallinen Hydroxyapatitpartikeln, besteht.

5. Biphasischer keramischer Knochenersatz nach einem der Ansprüche 1-4, wobei das knochenaktive Protein ausgewählt ist aus der Gruppe, umfassend knochenmorphogene Proteine (BMP), insulinähnliche Wachstumsfaktoren (IGF), transformierende Wachstumsfaktoren-β (TGFβ), Nebenschilddrüsenhormon (PTH), Sclerostin, aus Zellfabriken abgeleitete knochenaktive Proteine und extrazelluläre Matrix (ECM)-Proteine.

6. Biphasischer keramischer Knochenersatz nach Anspruch 5, wobei das knochenaktive Protein ein knochenmorphogenes Protein (BMP), zum Beispiel BMP-2, vorzugsweise rhBMP-2, und/oder BMP-7, vorzugsweise rhBMP-7, ist.

7. Biphasischer keramischer Knochenersatz nach einem der Ansprüche 1-6, wobei das antikatabolische Mittel ein Bisphosphonat ist, das ausgewählt ist aus der Gruppe, umfassend Etidronat, Clodronat und Tiludronat, oder aus der Gruppe, umfassend Pamidronat, Neridronat, Olpadronat, Alendronat, Ibandronat, Risedronat und Zoledronat.

8. Biphasischer keramischer Knochenersatz nach einem der Ansprüche 1-7, umfassend mindestens ein weiteres bioaktives Mittel, ausgewählt aus Antibiotika, Antimykotika, Knochenheilungspromotoren, Chemotherapeutika, Zytostatika, Vitaminen, Hormonen, Knochenmarkaspirat, thrombozytenreichem Plasma und demineralisiertem Knochen.

9. Biphasischer keramischer Knochenersatz nach Anspruch 8, umfassend mindestens ein Antibiotikum, ausgewählt aus Gentamycin, Vancomycin, Tobramycin, Cefazolin, Rifampicin, Clindamycin, und wobei das Antimykotikum ausgewählt ist aus der Gruppe, umfassend Nystatin, Griseofulvin, Amphotericin B, Ketoconazol und Miconazol.

10. Biphasischer keramischer Knochenersatz nach einem der Ansprüche 1-9, weiter umfassend ein Röntgenkontrastmittel, das aus wasserlöslichen nichtionischen Röntgenkontrastmitteln und/oder biologisch abbaubaren Röntgenkontrastmitteln ausgewählt ist.

11. Biphasischer keramischer Knochenersatz nach Anspruch 10, wobei das wasserlösliche nichtionische Röntgenkontrastmittel aus Iohexol, Iodixanol, Ioversol, Iopamidol, Iotrolan, Metrizamid, Iodecimol, Ioglucol, Ioglucamid, Ioglunid, Iogulamid, Iomeprol, Iopentol, Iopromid, Iosarcol, Iosimid, Iotusal, Ioxilan, Iofrotal und Iodecol ausgewählt ist.

12. Biphasischer keramischer Knochenersatz nach einem der Ansprüche 1-11, wobei das Verhältnis von Calciumsulfat zu Calciumphosphat (Gew./Gew.) 5 : 95 bis 95 : 5, 10 : 90 bis 90 : 10, 20 : 80 bis 80 : 20, 30 : 70 bis 70 : 30 oder 40 : 60 bis 60 : 40 beträgt.

13. Biphasische härtbare keramische Knochenersatzpaste zum Herstellen eines biphasischen keramischen Knochenersatzes nach einem der Ansprüche 1-12, umfassend:
a. ein Calciumsulfathemihydratpulver;
b. ein Calciumphosphatpulver, wobei das Calciumphosphat aus α-Tricalciumphosphat, Hydroxyapatit, Tetracalciumphosphat und β-Tricalciumphosphat ausgewählt ist;
c. ein knochenaktives Protein;
d. ein antikatabolisches Mittel, das die Knochenresorption hemmt, ausgewählt aus Bisphosphonsäuren und Bisphosphonaten;
e. wahlweise einen Beschleuniger zum Einstellen von Calciumsulfat in einer wässrigen Lösung, vorzugsweise aus Calciumsulfatdihydrat und einem anorganischen Salz, zum Beispiel NaCl, ausgewählt;
f. wahlweise einen Beschleuniger zum Einstellen von Calciumphosphat in einer wässrigen Lösung, vorzugsweise teilchenförmiges Calciumphosphat und/oder ein Phosphatsalz, zum Beispiel Na₂HPO₄; und
g. eine wässrige Flüssigkeit.

14. Biphasische härtbare keramische Knochenersatzpaste nach Anspruch 13, wobei das antikatabolische Mittel mit den Calciumphosphatpartikeln in dem Pulver vorgemischt und an sie gebunden ist.

15. Biphasische härtbare keramische Knochenersatzpaste nach Anspruch 13 oder Anspruch 14, wobei die Calciumphosphatpartikel kristalline Hydroxyapatitpartikel sind.

16. Biphasische härtbare keramische Knochenersatzpaste nach einem der Ansprüche 13-15, weiter umfassend ein bioaktives Mittel, ausgewählt aus Antibiotika, Antimykotika, Knochenheilungspromotoren, Chemotherapeutika, Zytostatika, Vitaminen, Hormonen, Knochenmarkaspirat, thrombozytenreichem Plasma und demineralisiertem Knochen.

17. Biphasische härtbare keramische Knochenersatzpaste nach einem der Ansprüche 13-16, weiter umfassend ein Röntgenkontrastmittel, das aus wasserlöslichen nichtionischen Röntgenkontrastmitteln und/oder biologisch abbaubaren Röntgenkontrastmitteln ausgewählt ist.

18. Biphasische härtbare keramische Knochenersatzpaste nach einem der Ansprüche 13-17, wobei das knochenaktive Protein und/oder das antikatabolische Mittel und/oder das weitere bioaktive Mittel und/oder das Röntgenkontrastmittel mit der wässrigen Flüssigkeit gemischt wird/werden, bevor es/sie mit dem Pulver/den Pulvern gemischt wird/werden.

19. Biphasische härtbare keramische Knochenersatzpaste nach einem der Ansprüche 13-18, wobei die Paste injizierbar ist.

20. Biphasische härtbare keramische Knochenersatzpaste nach einem der Ansprüche 13-19, wobei das Verhältnis von Calciumsulfat zu Calciumphosphat (Gew./Gew.) 5 : 95 bis 95 : 5, 10 : 90 bis 90 : 10, 20 : 80 bis 80 : 20, 30 : 70 bis 70 : 30 oder 40 : 60 bis 60 : 40 beträgt; und das Verhältnis von Flüssigkeit zu trockenem Pulvers 0,2 bis 0,8, vorzugsweise 0,3 bis 0,6, beträgt.

21. Biphasische härtbare keramische Knochenersatzpaste nach einem der Ansprüche 13-20 zur Verwendung bei der Behandlung einer Störung des Stützgewebes bei einem menschlichen oder nichtmenschlichen Subjekt, die eine anfängliche Stimulation von Knochenzellaktivierung und -wachstum und eine Unterdrückung von vorzeitiger Resorption des neu gebildeten Knochens einschließt.

22. Kit zum Herstellen einer biphasischen härtbaren keramischen Knochenersatzpaste nach einem der Ansprüche 13-20 oder eines biphasischen keramischen Knochenersatzes nach einem der Ansprüche 1-12, das die folgenden Komponenten umfasst:
i) ein Calciumsulfathemihydratpulver;
ii) ein Pulver eines Calciumphosphats nach Anspruch 3 oder Anspruch 4;
iii) ein knochenaktives Protein nach Anspruch 5 oder Anspruch 6;
iv) ein antikatabolisches Mittel nach Anspruch 7;
v) wahlweise mindestens ein weiteres bioaktives Mittel nach Anspruch 8 oder Anspruch 9;
vi) wahlweise ein Röntgenkontrastmittel nach Anspruch 10 oder Anspruch 11;
vii) wahlweise einen Beschleuniger zum Einstellen des Calciumsulfats, vorzugsweise Calciumsulfatdihydrat oder ein organisches Salz, zum Beispiel NaCl;
viii) wahlweise einen Beschleuniger zum Einstellen des Calciumphosphats, vorzugsweise teilchenförmiges Calciumphosphat und/oder ein Calciumphosphatsalz, zum Beispiel Na₂HPO₄; und
ix) wahlweise eine wässrige Flüssigkeit, zum Beispiel Wasser.

23. Kit nach Anspruch 22, weiter umfassend:
a. (eine) Misch- und/oder Injektionsvorrichtung(en) und/oder
b. Gebrauchsanweisungen.

24. Kit nach Anspruch 22 oder Anspruch 23, weiter umfassend eine biologisch abbaubare synthetische Membran oder eine Kollagenmembran.

25. Biphasischer keramischer Knochenersatz nach einem der Ansprüche 1-12, biphasische keramische Knochenersatzpaste nach einem der Ansprüche 13-21 oder Kit nach einem der Ansprüche 22-24, wobei eines oder mehrere der Additive einzeln oder in (einer) beliebigen Kombination(en) in wasserlöslichen und/oder biologisch abbaubaren synthetischen polymeren Mikrokapseln, Rinderkollagenpartikeln, Stärkepartikeln, Dihydratnidationspartikeln verkapselt ist/sind.

26. Biphasischer keramischer Knochenersatz nach einem der Ansprüche 1-12 und 25 oder biphasische keramische Knochenersatzpaste nach einem der Ansprüche 13-21 zur Verwendung bei der Behandlung eines Patienten mit einem Knochendefekt, zum Beispiel Knochenverlust infolge unter anderem eines Traumas, einer Eradikation einer Infektion, einer Resektion von Tumorläsionen, einer verzögerten oder ausbleibenden Knochenheilung, sowie bei primären oder Revisionsarthroplastiken, wobei die Behandlung das Einsetzen eines oder mehrerer biphasischer keramischer Knochenersätze (Implantate) oder der biphasischen härtbaren biphasischen keramischen Knochenersatzpaste anstelle des entfernten Knochens umfasst.

## Revendications

1. Substitut osseux céramique biphasique comprenant :
a. une phase de sulfate de calcium ;
b. une phase de phosphate de calcium ;
c. au moins une protéine active osseuse, et
d. au moins un agent anti-catabolique qui inhibe la résorption osseuse sélectionné à partir d'acides bisphosphoniques et de bisphosphonates,
dans lequel la au moins une protéine active osseuse est comprise dans la phase de sulfate de calcium et l'au moins un agent anti-catabolique est compris dans la phase de phosphate de calcium.

2. Substitut osseux céramique biphasique selon la revendication 1, dans lequel le sulfate de calcium est un dihydrate de sulfate de calcium.

3. Substitut osseux céramique biphasique selon la revendication 1 ou la revendication 2, dans lequel le phosphate de calcium est sélectionné à partir du groupe constitué par du phosphate α-tricalcique, de l'hydroxyapatite, du phosphate tétracalcique et du phosphate β-tricalcique.

4. Substitut osseux céramique biphasique selon la revendication 3, dans lequel la phase de phosphate de calcium est composée d'hydroxyapatite, de préférence de particules d'hydroxyapatite cristalline.

5. Substitut osseux céramique biphasique selon l'une quelconque des revendications 1 à 4, dans lequel la protéine active osseuse est sélectionnée à partir du groupe constitué par des protéines morphogénétiques osseuses (BMP), des facteurs de croissance analogues à l'insuline (IGF), des facteurs β de croissance transformants (TGFβ), une parathormone (PTH), une sclérostine, des protéines actives osseuses dérivées d'usine à cellules et de protéines de matrice extracellulaire (ECM).

6. Substitut osseux céramique biphasique selon la revendication 5, dans lequel la protéine active osseuse est une protéine morphogénétique osseuse (BMP), telle que BMP-2, de préférence rhBMP-2, et/ou BMP-7, de préférence rhBMP7.

7. Substitut osseux céramique biphasique selon l'une quelconque des revendications 1 à 6, dans lequel l'agent anti-catabolique est un bisphosphonate sélectionné à partir du groupe constitué par de l'étidronate, du clodronate et du tiludronate, ou du groupe constitué par du pamidronate, du néridronate, de l'olpadronate, de l'alendronate, de l'ibandronate, du risédronate et du zolédronate.

8. Substitut osseux céramique biphasique selon l'une quelconque des revendications 1 à 7, comprenant au moins un autre agent bioactif sélectionné à partir d'antibiotiques, de médicaments antifongiques, de promoteurs de cicatrisation osseuse, d'agents chimiothérapeutiques, de cytostatiques, de vitamines, d'hormones, d'aspirat de moelle osseuse, de plasma riche en plaquettes et d'os déminéralisé.

9. Substitut osseux céramique biphasique selon la revendication 8, comprenant au moins un antibiotique sélectionné à partir de gentamicine, de vancomycine, de tobramycine, de céfazoline, de rifampicine, de clindamycine et le médicament antifongique est sélectionné à partir du groupe constitué par de la nystatine, de la griséofulvine, de l'amphotéricine B, du cétoconazole et du miconazole.

10. Substitut osseux céramique biphasique selon l'une quelconque des revendications 1 à 9, comprenant en outre un agent de contraste aux rayons X sélectionné à partir d'agents de contraste aux rayons X non ioniques solubles dans l'eau et/ou d'agents de contraste aux rayons X biodégradables.

11. Substitut osseux céramique biphasique selon la revendication 10, dans lequel l'agent de contraste aux rayons X non ionique soluble dans l'eau est sélectionné à partir d'iohexol, d'iodixanol, d'ioversol, d'iopamidol, d'iotrolane, de métrizamide, d'iodécimol, d'ioglucol, d'ioglucamide, d'ioglunide, d'iogulamide, d'ioméprol, d'iopentol, d'iopromide, d'iosarcol, d'iosimide, d'iotusal, d'ioxilane, d'iofrotal, et d'iodécol.

12. Substitut osseux céramique biphasique selon l'une quelconque des revendications 1 à 11, dans lequel le rapport du sulfate de calcium au phosphate de calcium (p/p) est de 5 : 95 à 95 : 5, de 10 : 90 à 90 : 10, de 20 : 80 à 80 : 20, de 30 : 70 à 70 : 30, ou de 40 : 60 à 60 : 40.

13. Pâte de substitut osseux céramique durcissable biphasique pour produire un substitut osseux céramique biphasique selon l'une quelconque des revendications 1 à 12, comprenant :
a. une poudre hémihydratée de sulfate de calcium ;
b. une poudre de phosphate de calcium, où le phosphate de calcium est sélectionné à partir de phosphate α-tricalcique, d'hydroxyapatite, de phosphate tétracalcique et de phosphate β-tricalcique ;
c. une protéine active osseuse ;
d. un agent anti-catabolique qui inhibe la résorption osseuse sélectionné à partir d'acides bisphosphoniques et de bisphosphonates ;
e. éventuellement un accélérateur pour la prise de sulfate de calcium dans une solution aqueuse, de préférence sélectionné à partir de dihydrate de sulfate de calcium et d'un sel inorganique, tel que NaCl ;
f. éventuellement un accélérateur pour la prise de phosphate de calcium dans une solution aqueuse, de préférence un phosphate de calcium particulaire ; et/ou un sel de phosphate, tel que Na₂HPO₄ ; et
g. un liquide aqueux.

14. Pâte de substitut osseux céramique durcissable biphasique selon la revendication 13, dans laquelle l'agent anti-catabolique est prémélangé et lié aux particules de phosphate de calcium dans la poudre.

15. Pâte de substitut osseux céramique durcissable biphasique selon la revendication 13 ou la revendication 14, dans laquelle les particules de phosphate de calcium sont des particules d'hydroxyapatite cristalline.

16. Pâte de substitut osseux céramique durcissable biphasique selon l'une quelconque des revendications 13 à 15, comprenant en outre un agent bioactif sélectionné à partir d'antibiotiques, de médicaments antifongiques, de promoteurs de cicatrisation osseuse, d'agents chimiothérapeutiques, de cytostatiques, de vitamines, d'hormones, d'un aspirat de moelle osseuse, de plasma riche en plaquettes et d'os déminéralisé.

17. Pâte de substitut osseux céramique durcissable biphasique selon l'une quelconque des revendications 13 à 16, comprenant en outre un agent de contraste aux rayons X sélectionné à partir d'agents de contraste aux rayons X non ioniques solubles dans l'eau et/ou d'agents de contraste aux rayons X biodégradables.

18. Pâte de substitut osseux céramique durcissable biphasique selon l'une quelconque des revendications 13 à 17, dans laquelle la protéine active osseuse et/ou l'agent anti-catabolique et/ou l'autre agent bioactif et/ou l'agent de contraste aux rayons X est/sont mélangé(s) avec le liquide aqueux avant d'être mélangé(s) à la/aux poudre(s).

19. Pâte de substitut osseux céramique durcissable biphasique selon l'une quelconque des revendications 13 à 18, dans laquelle la pâte est injectable.

20. Pâte de substitut osseux céramique durcissable biphasique selon l'une quelconque des revendications 13 à 19, dans laquelle le rapport du sulfate de calcium au phosphate de calcium (p/p) est de 5 : 95 à 95 : 5, de 10 : 90 à 90 : 10, de 20 : 80 à 80 : 20, de 30 : 70 à 70 : 30, ou de 40 : 60 à 60 : 40 ; et le rapport du liquide à la poudre sèche est de 0,2 à 0,8, de préférence de 0,3 à 0,6.

21. Pâte de substitut osseux céramique durcissable biphasique selon l'une quelconque des revendications 13 à 20, pour une utilisation dans le traitement d'un trouble d'un tissu de support chez un sujet humain ou non humain impliquant une stimulation initiale d'activation et de croissance de cellule osseuse et une suppression d'une résorption prématurée de l'os néoformé.

22. Kit pour la production d'une pâte de substitut osseux céramique durcissable biphasique selon l'une quelconque des revendications 13 à 20, ou d'un substitut osseux céramique durcissable selon l'une quelconque des revendications 1 à 12, comprenant les composants suivants :
i) une poudre hémihydratée de sulfate de calcium ;
ii) une poudre de phosphate de calcium telle que définie dans la revendication 3 ou la revendication 4 ;
iii) une protéine active osseuse telle que définie dans la revendication 5 ou la revendication 6 ;
iv) un agent anti-catabolique tel que défini dans la revendication 7 ;
v) éventuellement au moins un autre agent bioactif tel que défini dans la revendication 8 ou la revendication 9 ;
vi) éventuellement un agent de contraste aux rayons X tel que défini dans la revendication 10 ou la revendication 11 ;
vii) éventuellement un accélérateur pour la prise du sulfate de calcium, de préférence du dihydrate de sulfate de calcium ou d'un sel inorganique, tel que NaCl ;
viii) éventuellement un accélérateur pour la prise de phosphate de calcium, de préférence un phosphate de calcium particulaire et/ou un sel de phosphate de calcium tel que Na₂HPO₄ ; et
ix) éventuellement un liquide aqueux, tel que l'eau.

23. Kit selon la revendication 22, comprenant en outre :
a. un/des dispositif(s) de mélange et/ou d'injection, et/ou
b. des instructions d'utilisation.

24. Kit selon la revendication 22 ou la revendication 23, comprenant en outre une membrane synthétique biodégradable ou une membrane de collagène.

25. Substitut osseux céramique biphasique selon l'une quelconque des revendications 1 à 12, pâte de substitut osseux céramique durcissable biphasique selon l'une quelconque des revendications 13 à 21 ou kit selon l'une quelconque des revendications 22 à 24, dans lequel/laquelle un ou plusieurs des additifs est/sont fourni(s) sous une forme encapsulée individuellement ou dans une/des combinaison(s) quelconque(s) dans des microcapsules solubles dans l'eau et/ou des microcapsules polymères synthétiques biodégradables, des particules de collagène bovin, des particules d'amidon, des particules de nidation dihydratées.

26. Substitut osseux céramique biphasique selon l'une quelconque des revendications 1 à 12 et 25, ou pâte de substitut osseux céramique biphasique selon l'une quelconque des revendications 13 à 21 pour une utilisation dans le traitement d'un patient ayant un défaut osseux, tel qu'une perte d'os due, entre autres à un traumatisme, l'éradication d'une infection, une résection de lésions tumorales, des réunions retardées ou défectueuses et dans des arthroplasties primaires ou de révision, ledit traitement comprenant une insertion d'un ou plusieurs substitut(s) osseux céramique(s) biphasique(s) (greffons) ou de ladite pâte de substitut osseux céramique biphasique durcissable à la place de l'os retiré.
